# EUROPEAN PATENT SPECIFICATION

(11) **EP 4 221 704 B1**
(45) Date of publication and mention of the grant of the patent: **24.09.2025**
(21) Application number: 21876583.2
(22) Date of filing: 01.10.2021
(51) Int. Cl.: A61K 9/20, A61K 31/397, A61K 31/41, A61K 31/4162, A61P 35/02

(54) **PHARMACEUTICAL FORMULATIONS FOR TREATING DISEASES MEDIATED BY KDM1A**
PHARMAZEUTISCHE FORMULIERUNGEN ZUR BEHANDLUNG VON KRANKHEITEN, DIE DURCH KDM1A VERMITTELT WERDEN
FORMULATIONS PHARMACEUTIQUES POUR LE TRAITEMENT DE MALADIES MÉDIÉES PAR KDM1A

(30) Priority: 01.10.2020 US 202063086353 P
(43) Date of publication of application: 09.08.2023
(73) Proprietor: Imago Biosciences Inc., San Carlos, CA 94070 (US)
(72) Inventor: TAPPER, Amy, San Carlos, California 94070 (US); CELATKA, Cassandra, San Carlos, California 94070 (US); SOULLIAC, Patricia, San Carlos, California 94070 (US); VED, Parag, San Carlos, California 94070 (US); VORA, Namrata, San Carlos, California 94070 (US)
(74) Representative: Merck Sharp & Dohme LLC
(86) International application number: PCT/US2021/053141
(87) International publication number: WO 2022/072811

(56) References cited:
- EP-B1- 1 067 927
- WO-A1-2015/170237
- WO-A1-2015/170237
- WO-A1-2016/130952
- WO-A1-2018/035259
- WO-A1-2018/035259
- WO-A1-2021/118996
- US-A1- 2016 237 043
- US-A1- 2019 070 172

## Description

This application claims the benefit of priority of U.S. Provisional Application No. 63/086,353, filed October 1, 2020.

Inhibiting the enzyme KDM1A (also known as lysine-specific demethylase 1, LSD1, Flavin-containing Amine Oxidase Domain-Containing Protein, AOF2, BRAF35-HDAC Complex Protein BHC110, FAD-Binding Protein BRAF35-HDAC Complex), may alter gene expression in cells sufficient to restore their proper physiologic function or that of the tissue, organ or the patient as a whole. This may be achieved either by enhancing transcription of a gene or genes that are pathologically silenced, e.g., as is the case in some cancer cells and heritable diseases, or decreasing transcription of a gene or genes participating in the pathological state. As such, inhibiting KDM1A would be useful for the treatment of diseases such as cancer and heritable diseases such as Wilson disease, cardiomyopathies, and hemoglobinopathies.

Numerous therapeutic agents have been identified that have the effect of altering gene expression acting either directly on proteins, generally enzymes, that alter chromatin states or indirectly. Though the precise mechanisms of their action have not all been fully elucidated, those mechanism can be inferred from our understanding of the protein complexes that participate in the activation of specific gene expression. These agents include 5'-azacytadine and 5'-aza -2' deoxycytidine (decitabine) which inhibit DNMT1 or other DNA methyltransferases known to be present and active at promoter sites of silenced genes such as gamma globin promoter; vorinostat and panobinostat or other inhibitors of histone deacetylase (HDAC) enzymes; hydroxyurea (HU), valproate and sodium butyrate and its analogues each of which may interfere with the activity of orphan nuclear receptors. All of these agents enjoy some clinical use principally in the management of neoplastic disease. Though some clinical utility of these agents for other disease states has been demonstrated, these agents have not been widely adopted because of their modest therapeutic effects and their toxicity.

The use of agents that inhibit any enzymatic activity resident in the protein complex bound to gene promoter has the potential to disrupt the repression of gamma globin gene expression and result in increased levels of fetal hemoglobin also known as hemoglobin F (HbF). Such targets include any of the interfaces of the specific protein-protein contacts, for example, the NuRD complex and KDM1A; the DNA binding recognition domains of, for example, NR2C1 and NR2C2; the ligand binding domains of, for example, NR2C1 and NR2C2; the enzyme activities such as lysine demethylase, for example, KDM1A; histone deacetylases (HDAC), for example HDAC1, 2, or 3; DNA methyltransferases, for example, DNMT1.

There remains a need for compositions and methods for altering gene expression in cells and tissues sufficient to restore the cell or tissue to normal physiologic function including, e.g., appropriate apoptosis in the case of cancer, or to alter the pathological phenotype of the cell, tissue, organ or organism by inducing the expression of one or more genes sufficiently to suppress the pathological state.

The compound N-((S)-5-((1R,2S)-2-(4-fluorophenyl)cyclopropylamino)-1-(4-methylpiperazin-1-yl)-1-oxopentan-2-yl)-4-(1H-1,2,3-triazol-1-yl)benzamide, herein referred to as Compound **A,** or Cpd **A,** has shown activity for the inhibition of KDM1A.

Pharmaceutically acceptable salts of Compound **A** have been prepared and examined. A ditosylate salt of Compound **A,** *N-*((*S*)-5-((1*R*,2*S*)-2-(4-fluorophenyl)cyclopropylamino)-1-(4-methylpiperazin-1-yl)-1-oxopentan-2-yl)-4-(1H-1,2,3-triazol-1-yl)benzamide ditosylate, herein referred to as Compound **B,** or Cpd **B,** has shown activity for the inhibition of KDM1A.

Accordingly, the inventors herein disclose new formulations and methods for treating diseases associated with KDM1A activity.

### Summary

Provided is a pharmaceutical composition comprising:
*N*-((*S*)-5-((1*R*,2*S*)-2-(4-fluorophenyl)cyclopropylamino)-1-(4-methylpiperazin-1-yl)-1-oxopentan-2-yl)-4-(1*H*-1,2,3-triazol-1-yl)benzamide (Compound **A),** or a pharmaceutically acceptable salt thereof, and
at least one stabilizer chosen from citric acid, fumaric acid, and tartaric acid.

Also provided is a pharmaceutical composition comprising:
a pharmaceutically acceptable salt of Compound **A,** and
at least one stabilizer chosen from citric acid, fumaric acid, and tartaric acid.

Also provided is a pharmaceutical composition comprising:
a tosylate salt of Compound **A,** and
at least one stabilizer chosen from citric acid, fumaric acid, and tartaric acid.

Also provided is a pharmaceutical composition comprising:
*N*-((*S*)-5-((1*R*,2*S*)-2-(4-fluorophenyl)cyclopropylamino)-1-(4-methylpiperazin-1-yl)-1-oxopentan-2-yl)-4-(1H-1,2,3-triazol-1-yl)benzamide ditosylate (Compound **B),** and
at least one stabilizer chosen from citric acid, fumaric acid, and tartaric acid.

Also provided is a pharmaceutical preparation comprising a formulation as described herein.

Also provided is a method of treating a disease or disorder associated with KDM1A activity in a patient in need thereof, the method comprising: administering to the patient in need thereof a therapeutically effective amount of a pharmaceutical composition described herein or a pharmaceutical preparation described herein.

Also provided is a method of inhibition of KDM1A comprising administering to the patient in need thereof a therapeutically effective amount of a pharmaceutical composition described herein or a pharmaceutical preparation described herein.

Also provided is a method for suppressing proliferation of malignant myeloid cells in a subject in need thereof, the method comprising administering to the patient in need thereof a therapeutically effective amount of a pharmaceutical composition described herein or a pharmaceutical preparation described herein.

These and other objects of the invention are described in the following paragraphs. These objects should not be deemed to narrow the scope of the invention.

### Brief Description of the Drawings

**FIG. 1** depicts a manufacturing process for 5 mg capsules of Compound **B** as described herein.
**FIG. 2** depicts a manufacturing process for 50 mg capsules of Compound **B** as described herein.
**FIG. 3** depicts trends in impurities (vertical axis) over 20 weeks for 5 mg formulations of Compound **B** in (a) white opaque capsules and (b) COLORISTA^{®} capsules.

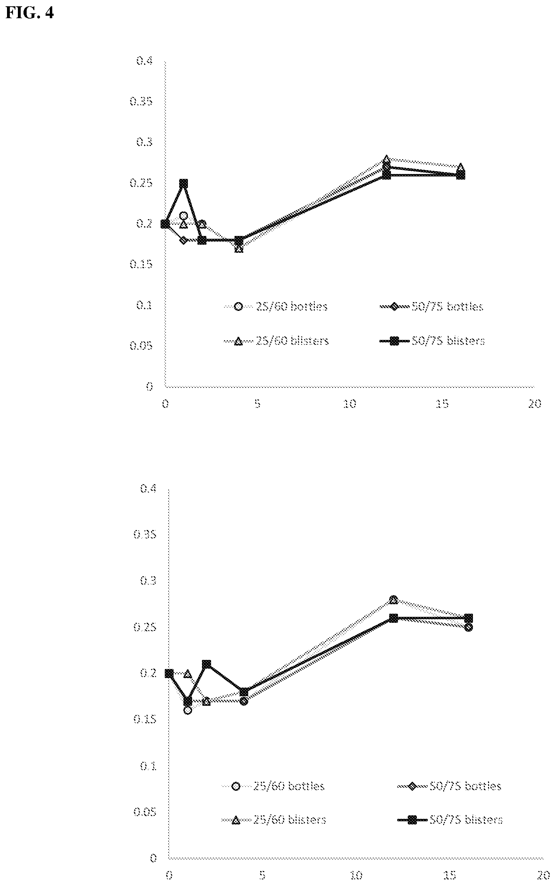

**FIG. 4** depicts trends in impurities (vertical axis) over 20 weeks for 50 mg formulations of Compound **B** in (a) white opaque capsules and (b) COLORISTA^{®} capsules.
**FIG. 5** depicts % release (vertical axis) as a function of time (min, horizontal axis) for 5 mg doses of Compound A in (a) white capsules with crospovidone, white capsules without crospovidone, (c) COLORISTA^{®} capsules with crospovidone, and (d) COLORISTA^{®} capsules without crospovidone.
**FIG. 6** depicts a manufacturing process for 5 mg capsules of Compound **B** as described herein.

### Detailed Description of the Invention

This detailed description is intended only to acquaint others skilled in the art with the present invention, its principles, and its practical application so that others skilled in the art may adapt and apply the invention in its numerous forms, as they may be best suited to the requirements of a particular use. This description and its specific examples are intended for purposes of illustration only. This invention, therefore, is not limited to the embodiments described in this patent application, and may be variously modified.

### Definitions

As used in the specification and the appended claims, unless specified to the contrary, the following terms have the meaning indicated:
The term "API" as used herein stands for "active pharmaceutical ingredient." The API as disclosed herein is *N*-((*S*)-5-((1*R*,2*S*)-2-(4-fluorophenyl)cyclopropylamino)-1-(4-methylpiperazin-1-yl)-1-oxopentan-2-yl)-4-(1*H*-1,2,3-triazol-1-yl)benzamide (Compound **A)** or a pharmaceutically acceptable salt thereof

As used herein, the term "pharmaceutical composition" means a composition comprising Compound **A** or a pharmaceutically acceptable salt thereof and, optionally, one or more pharmaceutically acceptable excipients.

The term "pharmaceutically acceptable" is used adjectivally to mean that the modified noun is appropriate for use as a pharmaceutical product for human use or as a part of a pharmaceutical product for human use.

The term "subject" includes humans and other primates as well as other mammals. In some embodiments, the subject is a human.

The term "therapeutically effective amount" means a sufficient amount of the API or pharmaceutical composition to treat a condition, disorder, or disease, at a reasonable benefit / risk ratio applicable to any medical treatment.

The terms "treat", "treating" and "treatment" refer to a method of alleviating or abrogating a condition, disorder, or disease and / or the attendant symptoms thereof.

The term "Cₘₐₓ" refers to the peak concentration and, in particular, the maximum observed plasma / serum concentration of drug.

The term "Tₘₐₓ" refers to the time to reach the peak concentration.

The term "AUCₜ" refers to the area under the plasma concentration-time curve, where t is the time of the last measurable plasma concentration in the study.

The term "AUC_{∞}" refers to the area under the plasma concentration-time curve from time zero to infinity following a single dose.

The term ""immediate release" pharmaceutical formulation includes any formulation in which the rate of release of drug from the formulation and / or the absorption of drug, is neither appreciably, nor intentionally, retarded by galenic manipulations. Thus, the term excludes formulations which are adapted to provide for "modified", "controlled", "sustained", "prolonged", "extended" or "delayed" release of drug. In this context, the term "release" includes the provision (or presentation) of drug from the formulation to the gastrointestinal tract, to body tissues and / or into systemic circulation.

As used herein, "about" means ± 20% of the stated value, and includes more specifically values of ± 10%, ± 5%, ± 2% and ± 1 % of the stated value.

### B. DRUG SUBSTANCE

Pharmaceutical compositions disclosed herein comprise at least one active pharmaceutical ingredient: N-((*S*)-5-((1R,2*S*)-2-(4-fluorophenyl)cyclopropylamino)-1-(4-methylpiperazin-1-yl)-1-oxopentan-2-yl)-4-(1*H*-1,2,3-triazol-1-yl)benzamide (Compound **A** or Cpd **A),** or a pharmaceutically acceptable salt thereof.

Compound **A** has the following formula:

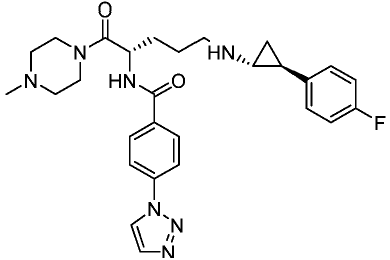

Methods of making Compound A and a pharmaceutically acceptable salt thereof are described in U.S. Patent No. 9,981,922.

Compound **A** may be present in a pharmaceutical composition in the form of acid addition salts. Acid addition salts of the free amino compounds may be prepared by methods well known in the art, and may be formed from organic and inorganic acids. Suitable organic acids include maleic, fumaric, benzoic, ascorbic, succinic, methanesulfonic, acetic, trifluoroacetic, oxalic, propionic, tartaric, salicylic, citric, gluconic, lactic, mandelic, cinnamic, aspartic, stearic, palmitic, glycolic, glutamic, p-toluenesulfonic acid, and benzenesulfonic acids. Suitable inorganic acids include hydrochloric, hydrobromic, sulfuric, phosphoric, and nitric acids. Thus, the term "pharmaceutically acceptable salt" of Compound **A** is intended to encompass any and all acceptable salt forms.

Certain pharmaceutical compositions disclosed herein comprise a ditosylate salt of Compound A, N-((S)-5-((1R,2S)-2-(4-fluorophenyl)cyclopropylamino)-1-(4-methylpiperazin-1-yl)-1-oxopentan-2-yl)-4-(1H-1,2,3-triazol-1-yl)benzamide ditosylate (Compound **B** or Cpd **B).**

Compound **B** has the following formula:

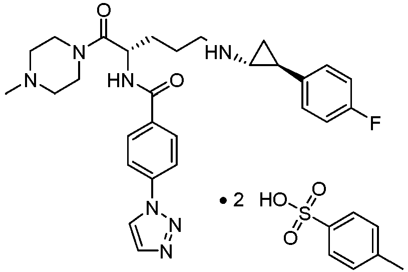

As used herein, and in the absence of a specific reference to a particular pharmaceutically acceptable salt of Compound **A,** any dosages, whether expressed in milligrams or as a percentage by weight or as a ratio with another ingredient, should be taken as referring to the amount of Compound **A.** For example, a reference to "20 mg Compound **A** or a pharmaceutically acceptable salt thereof" means an amount of Compound **A** or a pharmaceutically acceptable salt thereof that provides the same amount of Compound **A** as 20 mg of Compound **A** free form.

In some embodiments, Compound **A,** or a pharmaceutically acceptable salt thereof, is Compound **A** free base.

In some embodiments, Compound **A,** or a pharmaceutically acceptable salt thereof, is a pharmaceutically acceptable salt of Compound **A.**

In some embodiments, Compound **A,** or a pharmaceutically acceptable salt thereof, is a tosylate salt of Compound **A.** In some embodiments, Compound **A,** or a pharmaceutically acceptable salt thereof, is a ditosylate salt of Compound **A,** i.e., Compound **B.**

**In** some embodiments, the amount of Compound **A,** or pharmaceutically acceptable salt thereof, is from about 2 mg to about 100 mg. In some embodiments, the amount of Compound **A** is about 2.5, about 5, about 10, about 20, about 30, about 40, or about 50 mg. In some embodiments, the amount of Compound **A** is about 2.5, about 5, about 10, or about 20 mg. In some embodiments, the amount of Compound **A** is about 2.5 mg. In some embodiments, the amount of Compound **A** is about 5 mg. In some embodiments, the amount of Compound **A** is about 10 mg. In some embodiments, the amount of Compound **A** is about 20 mg. In some embodiments, the amount of Compound **A** is about 30 mg. In some embodiments, the amount of Compound **A** is about 40 mg. In some embodiments, the amount of Compound **A** is about 50 mg. In some embodiments, the amount of Compound **A** is about 60 mg. In some embodiments, the amount of Compound **A** is about 70 mg. In some embodiments, the amount of Compound **A** is about 80 mg. In some embodiments, the amount of Compound **A** is about 90 mg. In some embodiments, the amount of Compound **A** is about 100 mg.

In some embodiments, Compound **A,** or the pharmaceutically acceptable salt thereof, is present in an amount of between about 2 and about 10% w/w, measured as the free base. In some embodiments, Compound **A,** or the pharmaceutically acceptable salt thereof, is present in an amount of about 5% w/w, measured as the free base.

In some embodiments, Compound **A,** or the pharmaceutically acceptable salt thereof, is present in an amount of between about 20 and about 30% w/w, measured as the free base. In some embodiments, Compound **A,** or the pharmaceutically acceptable salt thereof, is present in an amount of about 25% w/w, measured as the free base.

### Pharmaceutical Compositions

This disclosure is directed to providing Compound **A** or a pharmaceutically acceptable salt thereof in a pharmaceutical composition that is pharmacologically efficacious and physically acceptable. The pharmaceutical compositions disclosed herein are intended for pharmaceutical use in human subjects.

Provided is a pharmaceutical composition comprising:
*N*-((S)-5-((1*R*,2*S*)-2-(4-fluorophenyl)cyclopropylamino)-1-(4-methylpiperazin-1-yl)-1-oxopentan-2-yl)-4-(1*H*-1,2,3-triazol-1-yl)benzamide (Compound **A),** or a pharmaceutically acceptable salt thereof, and at least one stabilizer chosen from citric acid, fumaric acid, and tartaric acid.

Also provided is a pharmaceutical composition comprising:
a pharmaceutically acceptable salt of Compound **A,** and
at least one stabilizer chosen from citric acid, fumaric acid, and tartaric acid.

Also provided is a pharmaceutical composition comprising:
a tosylate salt of Compound **A,** and
at least one stabilizer chosen from citric acid, fumaric acid, and tartaric acid.

Also provided is a pharmaceutical composition comprising:
*N*-((S)-5-((1*R*,2*S*)-2-(4-fluorophenyl)cyclopropylamino)-1-(4-methylpiperazin-1-yl)-1-oxopentan-2-yl)-4-(1H-1,2,3-triazol-1-yl)benzamide ditosylate (Compound **B),** and
at least one stabilizer chosen from citric acid, fumaric acid, and tartaric acid.

In some embodiments, at least one stabilizer is present in an amount of between about 2 and about 10% w/w. In some embodiments, at least one stabilizer is present in an amount of about 5% w/w.

In some embodiments, at least one stabilizer is present in an amount of between about 20 and about 30% w/w. In some embodiments at least one stabilizer is present in an amount of about 25% w/w.

In some embodiments, the composition comprises one or more fillers. In some embodiments, the one or more fillers is chosen from silicified microcrystalline cellulose, (PROSOLV^{®} SMCC HD 90), AVICEL^{®} dry granulation excipient (AVICEL^{®} DG), mannitol (PEARLITOL^{®} 200), anhydrous lactose, and pre-gelatinized starch (STARCH^{®}1500).

In some embodiments, the filler is anhydrous lactose.

In some embodiments, the filler is AVICEL^{®} DG.

In some embodiments, the filler is Starch 1500.

In some embodiments, the filler is a mixture of anhydrous lactose and AVICEL^{®} DG.

In some embodiments, the filler is present in the pharmaceutical composition in an amount of about 75 to about 90%. In some embodiments, the filler is present in the pharmaceutical composition in an amount of about 85%.

In some embodiments, the filler is present in the pharmaceutical composition in an amount of about 35 to about 50%. In some embodiments, the filler is present in the pharmaceutical composition in an amount of about 45%.

In some embodiments, the composition comprises one or more disintegrants. In some embodiments, the one or more disintegrants is chosen from croscarmellose sodium (AC-DI-SOL^{®}), Crospovidone XL (Polyplasdone^{™} XL), and sodium starch glycolate (EXPLOTAB^{®}). In some embodiments, the one or more disintegrants is POLYPLASDONE^{™} XL (crospovidone).

In some embodiments, the one or more disintegrant is present in the pharmaceutical composition in an amount between about 2 and about 10%. In some embodiments, the one or more disintegrant is present in the pharmaceutical composition in an amount about 5%.

In some embodiments, the composition comprises one or more lubricants. In certain further embodiments, the one or more lubricants is chosen from magnesium stearate (HYQUAL^{®}), sodium stearyl fumarate (PRUV^{®}), and stearic acid (GENAR^{®} Vegetable Grade, 50). In some embodiments, the one or more lubricants is magnesium stearate.

In some embodiments, the one or more lubricants is present in the pharmaceutical composition in an amount between about 0.1 and about 1%. In some embodiments, the one or more lubricants is present in the pharmaceutical composition in an amount about 0.5%.

In some embodiments, the composition comprises one or more binders. In certain further embodiments, the one or more binders is chosen from hypromellose (Methocel^{™} E3 Premium LV) and Povidone K-30 (KOLLIDON^{®} 30).

In some embodiments, the composition comprises one or more glidants. In certain further embodiments, the one or more glidants is chosen from colloidal silicon dioxide (CAB-O-SIL^{®}) and talc (Pharma 400 USP).

In some embodiments, the composition comprises a coating. In certain further embodiments, the coating is polyvinyl alcohol, part hydrolyzed polymer system (OPADRY^{®} Amb II).

In some embodiments, the composition is formulated with a direct blend. In some embodiments, the composition is formulated with a wet-granulation blend.

In some embodiments, the composition comprises:

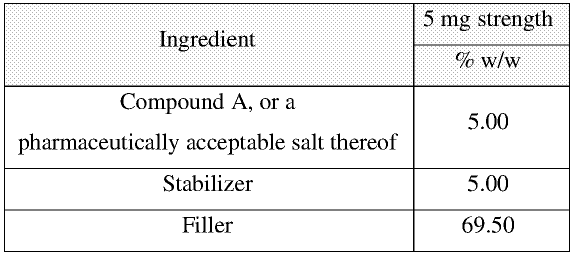

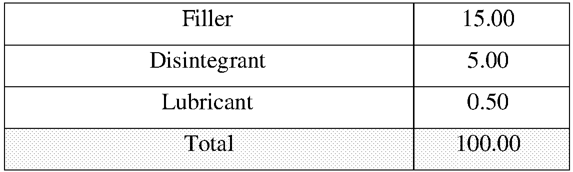

wherein Compound A is measured as the free base.

In some embodiments, the composition comprises:

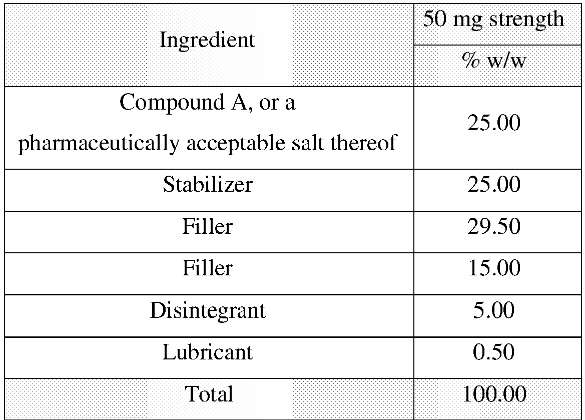

wherein Compound A is measured as the free base.

In some embodiments, the composition comprises:

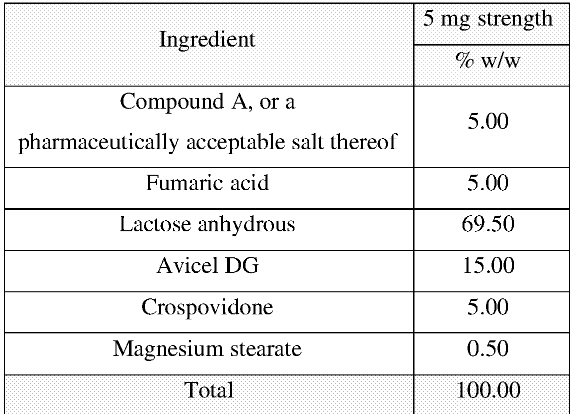

wherein Compound A is measured as the free base.

In some embodiments, the composition comprises:

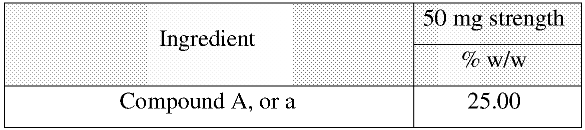

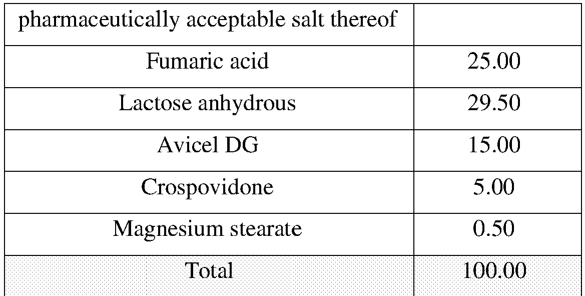

wherein Compound A is measured as the free base.

In some embodiments, the composition comprises:

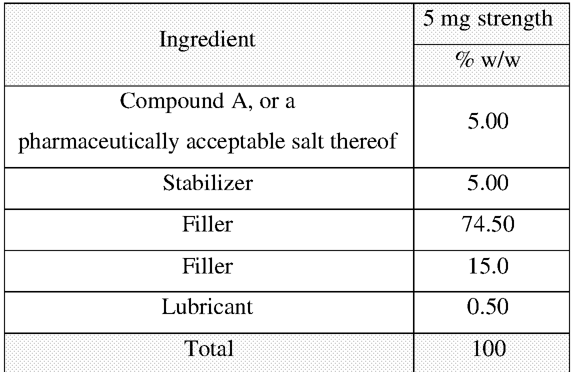

wherein Compound A is measured as the free base.

In some embodiments, the composition comprises:

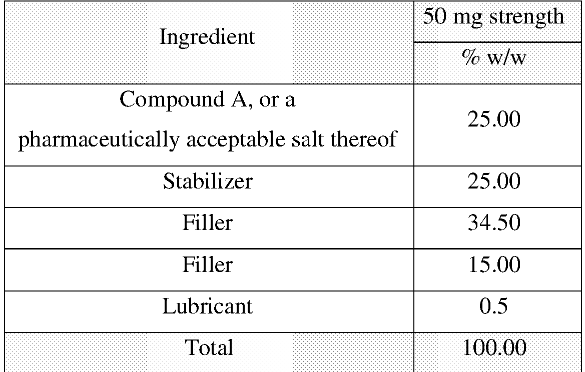

wherein Compound A is measured as the free base.

In some embodiments, the composition comprises:

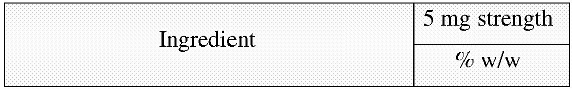

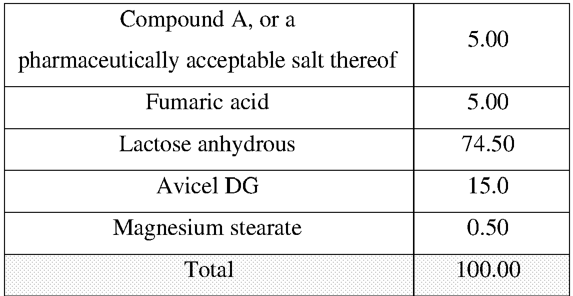

wherein Compound A is measured as the free base.

In some embodiments, the composition comprises:

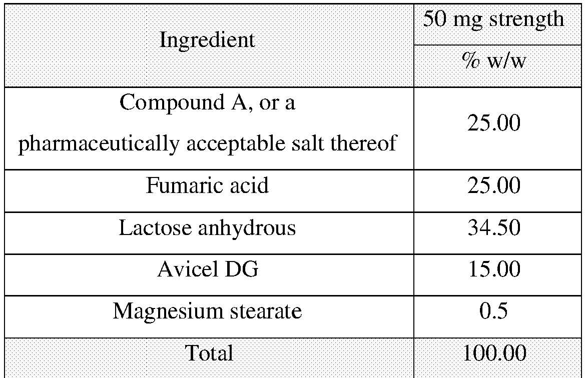

wherein Compound A is measured as the free base.

In some embodiments, the pharmaceutical compositions disclosed herein are stable during, for example, storage, distribution, and the duration of the product's shelf-life *(e.g.,* up to two years at room temperature / ambient conditions). A stable pharmaceutical composition may, for example, exhibit less degradation of the API and / or lower amounts of degradation products. Degradation products that arise during storage of the drug substance and / or drug product are undesirable and, in extreme cases, might even be harmful to a patient being treated with such drug product. Thus, it is desirable to control the formation of degradation products, particularly potentially harmful impurities in the drug product.

Assay and degradation product determination of pharmaceutical compositions may be performed using HPLC with UV detection. Assay and degradation product determination of pharmaceutical compositions may be performed using GC or GC/MS detection.

Pharmaceutical compositions may be assessed for degradation products following storage for at least two weeks, at least one month, at least two months, at least three months, at least six months, at least twelve months, at least eighteen months, or at least twenty four months. In particular, degradation products may be assessed at time intervals of one, three, six, nine, twelve, eighteen, twenty four, thirty six, and / or forty eight months. Storage conditions may be long term, intermediate, or accelerated conditions. In particular, storage conditions may be, for example, 25°C ± 2°C / 40% relative humidity (RH) ± 5% RH, 25°C ± 2°C / 60% RH ± 5% RH, 30°C ± 2°C / 35% RH ± 5% RH, 30°C ± 2°C / 65% RH ± 5% RH, 40°C ± 2°C / 25% RH ± 5% RH, 40°C ± 2°C / 75% RH ± 5% RH, 50°C ± 2°C / 75% RH ± 5% RH, 60°C ± 2°C / 5% RH ± 5% RH, 60°C ± 2°C / 40% RH ± 5% RH, 70°C ± 2°C / 5% RH ± 5% RH, 70°C ± 2°C / 75% RH ± 5% RH, and / or 80°C ± 2°C / 40% RH ± 5% RH.

### Pharmaceutical Preparations

While it may be possible for the compounds disclosed herein to be administered as the raw chemical, it is also possible to present them as pharmaceutical preparation.

Provided is a pharmaceutical preparation comprising a formulation as disclosed herein.

In some embodiments, the pharmaceutical preparation is a tablet. In some embodiments, the pharmaceutical preparation is a capsule. In some embodiments, the capsule is a COLORISTA^{®} capsule.

Pharmaceutical preparations that can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated to provide delayed, slowed, or controlled release or absorption of the active ingredient therein. Compositions may further comprise an agent that enhances solubility or dispersability. All formulations for oral administration should be in dosages suitable for such administration. The push-fit capsules can contain the active ingredients in admixture with filler such as lactose, binders such as starches, and / or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and / or titanium dioxide, lacquer solutions, and suitable organic solvents or solvent mixtures. Dyestuffs or pigments may be added to the tablets or dragee coatings for identification or to characterize different combinations of active compound doses.

### Methods of Use

Provided herein is a method of treating a disease or disorder associated with KDM1A activity, the method comprising administering a pharmaceutical composition or pharmaceutical preparation as described herein to a patient in need thereof.

In some embodiments, the disease or disorder is cancer.

In some embodiments, the disease is an inflammatory disease. In certain further embodiments, the inflammatory disease is chosen from inflammatory bowel disease, rheumatoid arthritis, or systemic lupus erythematosus.

In some embodiments, the disease or disorder is chosen from sickle cell disease, thalassemia major, and other beta-hemoglobinopathies

In some embodiments, the disease or disorder is a globin-mediated disease.

In some embodiments, the disease or disorder is a myeloproliferative neoplasm. In certain further embodiments, the myeloproliferative neoplasm is chosen from myelofibrosis, polycythemia vera, essential thrombocythemia, myelodysplastic syndrome (MDS), acute myelogenous leukemia (AML), and chronic myelogenous leukemia (CML). In certain further embodiments, the myelofibrosis is chosen from primary myelofibrosis and post -PV / ET myelofibrosis (PPV-MF and PET-MF).

Provided herein is a method for treating or preventing a myeloproliferative neoplasm in a subject in need thereof, the method comprising administering a pharmaceutical composition or pharmaceutical preparation as described herein to a patient in need thereof.

Further provided is a method for suppressing proliferation of malignant myeloid cells in a subject in need thereof, the method comprising administering a pharmaceutical composition or pharmaceutical preparation as described herein to a patient in need thereof.

Also provided is a method for reducing reticulin and collagen bone marrow fibrosis in a subject in need thereof, the method comprising administering a pharmaceutical composition or pharmaceutical preparation as described herein to a patient in need thereof.

Also provided is a method for reducing plasma levels of one or more inflammatory cytokines in a subject in need thereof, the method comprising administering a pharmaceutical composition or pharmaceutical preparation as described herein to a patient in need thereof.

Also provided is a method for reducing the mass of malignant myeloid cells in a subject in need thereof, the method comprising administering a pharmaceutical composition or pharmaceutical preparation as described herein to a patient in need thereof.

Also provided is a method for reducing abnormal spleen size or volume in a subject in need thereof, the method comprising administering a pharmaceutical composition or pharmaceutical preparation as described herein to a patient in need thereof.

Also provided is a method for reducing the amount of extramedullary hematopoiesis in a subject in need thereof, the method comprising administering a pharmaceutical composition or pharmaceutical preparation as described herein to a patient in need thereof.

Also provided is a method for reducing the constitutional symptoms of myelofibrosis measured by patient-reported surveys in a subject in need thereof, the method comprising administering a pharmaceutical composition or pharmaceutical preparation as described herein to a patient in need thereof.

Also provided is a method for reducing platelet counts in a subject in need thereof, the method comprising administering a pharmaceutical composition or pharmaceutical preparation as described herein to a patient in need thereof.

Also provided is a method for reducing bone marrow cellularity to age-adjusted normocellularity with fewer than 5% blast cells in a subject in need thereof, the method comprising administering a pharmaceutical composition or pharmaceutical preparation as described herein to a patient in need thereof.

Also provided is a method for a) reducing hemoglobin level in a PV patient to < 160 g / L, or b) decreasing red cell mass in a PV patient, wherein the decrease is inferred from hemoglobin levels Hb of < 160g / L, either method comprising administering a pharmaceutical composition or pharmaceutical preparation as described herein to a patient in need thereof.

Also provided is a method for increasing hemoglobin to >100 g / L in a MF patient, comprising administering a pharmaceutical composition or pharmaceutical preparation as described herein. Also provided is a method for increasing hemoglobin to a value >100 g / L and less than the upper limit of age-and sex adjusted normal in a subject in need thereof, the method comprising administering a pharmaceutical composition or pharmaceutical preparation as described herein to a patient in need thereof.

Also provided is a method for achieving an effect in a patient, the method comprising administering a pharmaceutical composition or pharmaceutical preparation as described herein, wherein the effect is chosen from an elevation of red blood cell count, an elevation of the red blood cell count of red cells containing fetal hemoglobin, an elevation in the total concentration of fetal hemoglobin in red cells, an elevation in the total concentration of fetal hemoglobin in reticulocytes, an increase in the transcription of the gamma globin gene in bone marrow-derived red cell precursors, e.g., pro-erythroblasts, a reduction in the number of sickle cell crises a patient experiences over a unit period of time, a halt to or prevention of tissue damage e.g. in the heart, spleen, brain or kidney caused by sickling cells, a reduction in the proportion of red cells that undergo sickling under physiological conditions of relative hypoxia as measured using patient blood in an *in vitro* assay, an increase in the amount of histone 3 lysine methylation at lysine position 4 (H3K4me1 and H3K4me2), and / or a decrease in the amount of histone 3 methylation at lysine position 9 (H3K9me1 or H3K4me2) near or at the gamma globin promoter as assayed by ChIP using cells derived from a treated patient.

Also provided herein is a method of inhibition of KDM1A, the method comprising administering to the patient in need thereof a therapeutically effective amount of a pharmaceutical composition or pharmaceutical preparation as disclosed herein.

Also provided is a method of inhibiting at least one KDM1A function, the method comprising administering a pharmaceutical composition or pharmaceutical preparation as described herein, wherein the inhibition is measured by phenotype of red cells or their precursors either cultured or *in vivo* in humans or mouse or transgenic mice containing the human beta globin locus or portions thereof, the ability of cancer cells to proliferate, the expression of specific genes known to be regulated by KDM1A activity such as gamma globin, a change in the histone methylation states, a change in the methylation state of proteins known to be demethylated by KDM1A such as G9a or SUV39H1, expression of KDM1A-regulated genes, or binding of KDM1A with a natural binding partner such as CoREST, DNMT1 or HDACs.

### Abbreviations

API = active pharmaceutical ingredient; HDAC = histone deacetylase; KDM1A = LC = loading capacity; LSD1 = lysine-specific demethylase 1; RRT = relative retention time; RS = related substance

### Examples

### Example 1. Initial studies of excipients / stabilizers.

Phase one of Compound **B** drug product development was initiated with an excipient compatibility study to identify excipients physically and chemically compatible with Compound **B** API. Commonly used excipients for oral solid dosage formulations were evaluated for this study including fillers, binders, disintegrants, glidants, lubricants and organic acid stabilizers.

**Table 1. Excipient list and ratios.**

| **Functionality** | **Material** | **Trade Name** | **Grade** | **Source** | **API : Excipient** |
|---|---|---|---|---|---|
| Fillers | Silicified microcrystalline cellulose | Prosolv SMCC HD 90 | NF, Ph. Eur., JP | JRS Pharma | 1 : 10 |
| | Avicel dry granulation excipient | Avicel DG | NF, Ph. Eur., JP | FMC | 1 : 10 |
| | Mannitol | Pearlitol^{®} 200 | USP, EP | Roquette | 1 : 10 |
| | Lactose Anhydrous | Lactose Anhydrous DT | NF | Kerry | 1 : 10 |
| | Pre-gelatinized starch | Starch 1500 | NF, Ph. Eur., | Colorcon | 1:10 |
| Stabilizers | Citric Acid powder, Anhydrous | NA | Multi-Compendial | Avantor Performance Materials | 1 : 1 |
| | Fumaric Acid | NA | Reagent Grade | Alfa Aesar^{™} | 1 : 1 |
| | Tartaric Acid | L-(+)-Tartaric Acid, Granular | ACS | Alfa Aesar^{™} | 1 : 1 |
| Binders | Hypromellose | Methocel E3 Premium LV | NF, EP, JP | Dow Chemical | 1 : 1 |
| | Povidone K-30 | Kollidon 30 | USP. Ph. EUR., JP | BASF | 1 : 1 |
| Disintegrant | Croscarmellose Sodium | Ac-Di-Sol | USP/NF | FMC | 1 : 1 |
| | Crospovidone XL | Polyplasdone XL | EP, USP | Ashland | 1 : 1 |
| | Sodium starch glycolate | Explotab | NF, Ph. EUR., JP | JRS Pharma | 1 : 1 |
| Glidant | Colloidal Silicon Dioxide | Cab-O-Sil | NF, EP, JP | Cabot | 1 : 0.1 |
| | Talc | Pharma 400 USP | USP | IMERYS | 1 : 0.1 |
| Lubricant | Magnesium Stearate | Hyqual | BP, JP, EP, NF | Mallinckrodt / Macron Fine Chemicals | 1 : 0.1 |
| | Sodium Stearyl Fumarate | Pruv | NF, Ph. Eur., JP, | JRS Pharma | 1 : 0.1 |
| | Stearic Acid (GenAR Vegetable Grade, 50) | NA | NF | Macron Fine Chemicals^{™} | 1 : 0.1 |
| Coating | Polyvinyl alycohol, part hydrolyzed polymer system | Opadry^{®} AMB II High Performance Moisture barrier coating 88A105052 blue | NA | Colorcon | 1 : 1 |

Samples were prepared with a binary mixture of Compound **B** and an excipients from Table **1.** Briefly, each excipient and API were individually weighed and filled in vials, followed by the addition of glass beads. The samples were closed, vortexed, and refrigerated at 2 to 8 °C prior to initiation of study as the sample preparation was executed over a period of 10 days. All vials were removed from the refrigerator and allowed to equilibrate to room temperature, then opened, and stored at the designated storage conditions at the same t = 0.

The sample of Compound **B** API and excipients were stored as binary mixtures in open vials at 50°C / 11% RH and 50°C / 75% RH conditions and evaluated at t = 0, 1 week, and 2 weeks for appearance, assay, and RS.

Stability data for individual compositions is presented in Tables **2 - 21.** A summary of % LC for Compound **B** in various formulations is presented in Table **22.**

**Table 2. Stability data of Compound B only.**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0935 | 0.1652 | 0.1453 | 0.3516 | 0.3545 |
| 1.022 | | | | 0.0489 | |
| 1.081 | 0.0589 | 0.0593 | 0.0649 | 0.0590 | 0.0715 |
| Total¹ | 0.1525 | 0.2245 | 0.2102 | 0.4595 | 0.4260 |

**Table 3. Stability data of Compound B with SMCC HD 90**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.1049 | 0.2288 | 0.3447 | 0.3125 | 0.8524 |
| 1.021 | 0.1359 | 0.0579 | 0.0732 | 0.0597 | 0.1225 |
| 1.081 | 0.0547 | 0.0574 | 0.0665 | 0.0589 | 0.0699 |
| 1.163 | | | | | 0.0527 |
| 1.674 | 0.0631 | | | | 0.0541 |
| Total¹ | 0.3586 | 0.3441 | 0.4844 | 0.4310 | 1.1516 |

**Table 4. Stability data of Compound B with Avicel DG**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.1060 | 0.2191 | 0.1894 | 0.3141 | 0.3421 |
| 1.022 | | 0.0431 | | 0.0776 | 0.0354 |
| 1.081 | 0.0595 | 0.0580 | 0.0633 | 0.0643 | 0.0674 |
| 1.674 | | | | 0.0339 | |
| Total¹ | 0.1655 | 0.3202 | 0.2527 | 0.4900 | 0.4449 |

**Table 5. Stability data of Compound B with Pearlitol 200**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0928 | 0.1412 | 0.1101 | 0.1851 | 0.2215 |
| 1.022 | 0.0522 | | | 0.0410 | |
| 1.081 | 0.0608 | 0.0625 | 0.0645 | 0.0599 | 0.0663 |
| Total¹ | 0.2058 | 0.2037 | 0.1746 | 0.2860 | 0.2878 |

**Table 6. Stability data of Compound B with Lactose anhydrous DT**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0996 | 0.1484 | 0.1106 | 0.2007 | 0.1314 |
| 1.021 | | | | 0.0767 | |
| 1.081 | 0.0604 | 0.0640 | 0.0614 | 0.0654 | 0.0666 |
| Total¹ | 0.1599 | 0.2124 | 0.1720 | 0.3428 | 0.1981 |

**Table 7. Stability data of Compound B with Starch 1500**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0969 | 0.1842 | 0.3354 | 0.2427 | 0.5712 |
| 1.081 | 0.0563 | 0.0552 | 0.0587 | 0.0606 | 0.0661 |
| 1.163 | | | | | 0.0392 |
| 1.669 | | | | | 0.0324 |
| 1.835 | | | | | 0.0426 |
| Total¹ | 0.1533 | 0.2395 | 0.3941 | 0.3033 | 0.7515 |

**Table 8. Stability data of Compound B with Citric Acid**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| 0.166 | | | 0.0533 | | 0.2171 |
| RS-1 | 0.1038 | 0.1878 | 0.0963 | 0.2399 | 0.1138 |
| 0.628 | | | | | 0.0650 |
| 0.837 | | | 0.1078 | | 0.2331 |
| 1.080 | 0.0571 | 0.0595 | 0.0433 | 0.0593 | 0.0389 |
| 1.101 | | | 1.3543 | | 2.2651 |
| 1.122 | | | 16.0487 | | 26.2109 |
| 1.163 | | | | | 0.0431 |
| 1.776 | | | 0.0611 | | 0.1719 |
| 1.785 | | | 0.7484 | | 2.1422 |
| Total¹ | 0.1609 | 0.2473 | 18.5132 | 0.2992 | 31.5010 |

**Table 9. Stability data of Compound B with Fumaric acid**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.1041 | 0.1490 | 0.0949 | 0.1642 | 0.0942 |
| 1.081 | 0.0585 | 0.0480 | 0.0539 | 0.0513 | 0.0562 |
| Total¹ | 0.1626 | 0.1970 | 0.1488 | 0.2155 | 0.1504 |

**Table 10. Stability data of Compound B with Tartaric Acid**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| 0.166 | | | 0.1385 | | 0.3568 |
| RS-1 | 0.1048 | 0.1872 | 0.1675 | 0.2275 | 0.1978 |
| 0.628 | | | 0.0478 | | 0.1576 |
| 0.637 | | | | | 0.0347 |
| 0.836 | | | 0.1467 | | 0.3472 |
| 0.966 | | | | | 0.0327 |
| 1.060 | | | 0.3192 | | 0.2105 |
| 1.072 | | | 2.5501 | | 1.7619 |
| 1.082 | 0.0624 | 0.0590 | | 0.0672 | |
| 1.101 | | | 1.5421 | | 3.1050 |
| 1.121 | | | 18.0858 | | 33.2692 |
| 1.162 | | | | | 0.0347 |
| 1.775 | | | 0.0651 | | 0.2025 |
| 1.784 | | | 0.7578 | | 2.3295 |
| Total¹ | 0.1672 | 0.2461 | 23.8208 | 0.2948 | 42.0401 |

**Table 11. Stability data of Compound B with Methocel E3 Premium LV**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0782 | 0.1411 | 2.4203 | 0.2391 | 2.8987 |
| 0.657 | | | 0.0372 | | 0.0429 |
| 0.712 | 0.0326 | | | | |
| 1.022 | | | | 0.0660 | |
| 0.798 | | | 0.0337 | | 0.0528 |
| 1.080 | 0.0610 | 0.0619 | 0.0776 | 0.0593 | 0.0857 |
| 1.163 | | | | | 0.0500 |
| 1.669 | | | 0.0698 | | 0.1414 |
| 1.674 | | | | 0.0306 | |
| 1.694 | | | 0.0481 | | 0.0770 |
| 1.867 | | | 0.0427 | | 0.0704 |
| Total¹ | 0.1718 | 0.2030 | 2.7295 | 0.3950 | 3.4190 |

**Table 12. Stability data of Compound B with Povidone K30**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| 0.166 | | | | | 0.0362 |
| RS-1 | 0.0999 | 0.2049 | 1.5751 | 0.2407 | 3.8513 |
| 0.657 | | | 0.0327 | | 0.1174 |
| 0.795 | | | | | 0.0431 |
| 1.021 | 0.0399 | 0.0446 | 0.4369 | 0.0687 | 0.6190 |
| 1.080 | 0.0596 | 0.0673 | 0.0833 | 0.0623 | 0.1131 |
| 1.163 | | | | | 0.0301 |
| 1.835 | | | 0.0331 | | 0.0506 |
| Total¹ | 0.1994 | 0.3167 | 2.1610 | 0.3717 | 4.8607 |

**Table 13. Stability data of Compound B with Croscarmellose sodium³**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | | | | | 0.2385 |
| 1.019 | 0.2552 | 0.0652 | 0.2504 | 0.1483 | 0.1764 |
| 1.052 | 0.2843 | 0.2345 | 0.2325 | 0.2087 | 0.1921 |
| 1.868 | | | | | 0.2251 |
| 1.931 | | | | | 0.1800 |
| Total¹ | 0.5395 | 0.2997 | 0.4828 | 0.3570 | 1.0121 |

**Table 14. Stability data of Compound B with Crospovidone**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0991 | 0.2044 | 0.3873 | 0.2344 | 0.8837 |
| 0.658 | | | | | 0.0368 |
| 1.021 | | 0.0499 | 0.1246 | 0.0471 | 0.1934 |
| 1.080 | 0.0597 | 0.0601 | 0.0619 | 0.0653 | 0.0657 |
| 1.163 | | | | | 0.0329 |
| Total¹ | 0.1588 | 0.3144 | 0.5738 | 0.3467 | 1.2126 |

**Table 15. Stability data of Compound B with sodium starch glycolate³**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| 0.561 | | | | | 0.0413 |
| RS-1 | 0.0978 | 0.2005 | 11.8015 | 0.2199 | 23.3999 |
| 0.628 | | | | | 0.0453 |
| 0.657 | | | 0.7556 | | 2.3693 |
| 0.700 | | | 0.0701 | | 0.1347 |
| 0.722 | | | | | 0.0419 |
| 0.798 | | | 0.2115 | | 0.6516 |
| 0.936 | | | 0.0421 | | 0.0487 |
| 1.020 | 0.0388 | 0.0420 | 0.1436 | 0.0752 | 0.1899 |
| 1.053 | 0.0346 | 0.0304 | | 0.0307 | |
| 1.078 | 0.0345 | 0.0333 | 0.1843 | 0.0352 | 0.3374 |
| 1.109 | | | | | 0.0489 |
| 1.116 | | | | | 0.0548 |
| 1.535 | | | | | 0.0410 |
| 1.606 | | | | | 0.0427 |
| 1.667 | | | 0.1042 | | 0.2943 |
| 1.692 | | | 0.1156 | | 0.2175 |
| 1.705 | | | | | 0.0317 |
| 1.741 | | | 0.0301 | | 0.0423 |
| 1.782 | | | 0.0515 | | 0.1052 |
| 1.826 | | | 0.0412 | | 0.1014 |
| 1.833 | | | 0.1338 | | 0.1722 |
| 1.847 | | | | | 0.0602 |
| 1.865 | | | 0.8949 | | 2.4785 |
| 1.878 | | | 0.0527 | | 0.5305 |
| 1.881 | | | 0.0632 | | |
| 1.888 | | | 0.6393 | | 1.4322 |
| 1.899 | | | | | 0.2936 |
| 1.909 | | | | | 0.0879 |
| 1.967 | | | | | 0.0542 |
| 1.982 | | | | | 0.1604 |
| 2.004 | | | | | 0.0356 |
| 2.051 | | | | | 0.1487 |
| Total¹ | 0.2058 | 0.3062 | 15.3350 | 0.3610 | 33.6939 |

**Table 16. Stability data of Compound B with Colloidal Silicon Dioxide**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0909 | 0.1952 | 0.1157 | 0.2536 | 0.2938 |
| 1.022 | 0.0495 | | | | |
| 1.082 | 0.0622 | 0.0597 | 0.0662 | 0.0672 | 0.0694 |
| 1.163 | | | | | 0.0304 |
| Total¹ | 0.2026 | 0.2548 | 0.1819 | 0.3208 | 0.3937 |

**Table 17. Stability data of Compound B with Talc**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0985 | 0.1838 | 0.2128 | 0.2240 | 0.2989 |
| 1.022 | | | | 0.0516 | |
| 1.081 | 0.0592 | 0.0593 | 0.0656 | 0.0603 | 0.0732 |
| Total¹ | 0.1577 | 0.2431 | 0.2784 | 0.3358 | 0.3721 |

**Table 18. Stability data of Compound B with Magnesium Stearate**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.1026 | 0.1900 | 0.2694 | 0.2545 | 0.4371 |
| 1.022 | | | 0.0746 | 0.0459 | 0.0436 |
| 1.081 | 0.0607 | 0.0627 | 0.0649 | 0.0657 | 0.0700 |
| 1.869 | | | | | 0.0306 |
| Total¹ | 0.1634 | 0.2527 | 0.4089 | 0.3660 | 0.5813 |

**Table 19. Stability data of Compound B with Sodium stearyl fumarate**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0947 | 0.2203 | 0.2226 | 0.2574 | 0.3665 |
| 0.795 | | | | | 0.0406 |
| 1.022 | 0.0405 | | 0.0534 | 0.0701 | 0.0481 |
| 1.081 | 0.0592 | 0.0616 | 0.0634 | 0.0590 | 0.0710 |
| 1.868 | | | | | 0.0317 |
| Total¹ | 0.1945 | 0.2819 | 0.3394 | 0.3865 | 0.5580 |

**Table 20. Stability data of Compound B with Stearic Acid 50 Vegetable grade**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.0908 | 0.1121 | 0.1119 | 0.1306 | 0.1107 |
| 1.022 | 0.0441 | | | 0.0426 | |
| 1.082 | 0.0580 | 0.0599 | 0.0641 | 0.0648 | 0.0663 |
| Total¹ | 0.1929 | 0.1721 | 0.1760 | 0.2379 | 0.1771 |

**Table 21. Stability data of Compound B with Opadry AMB II Blue**

| **Time, week** | 0 | 1 | 1 | 2 | 2 |
|---|---|---|---|---|---|
| **RH** | | 11 % | 75 % | 11 % | 75 % |
| **Name / RRT** | | | | | |
| RS-1 | 0.1059 | 0.1520 | 5.0623 | 0.1947 | 7.4602 |
| 0.657 | | | 0.2100 | | 0.5264 |
| 0.701 | | | 0.0450 | | 0.0890 |
| 0.722 | | | | | 0.0540 |
| 0.799 | | | 0.0661 | | 0.1375 |
| 0.937 | | | | | 0.0447 |
| 0.965 | | | | | 0.0327 |
| 1.021 | 0.0911 | 0.0894 | 0.1555 | 0.0951 | 0.1396 |
| 1.080 | 0.0547 | 0.0585 | 0.0936 | 0.0639 | 0.1076 |
| 1.669 | | | 0.1557 | | 0.3814 |
| 1.694 | | | 0.1109 | | 0.2010 |
| 1.784 | | | 0.0323 | | 0.0515 |
| 1.835 | | | 0.1696 | | 0.2694 |
| 1.867 | | | 0.1551 | | 0.3655 |
| 1.890 | | | 0.1093 | | 0.2835 |
| 1.933 | | | | | 0.0307 |
| Total¹ | 0.2517 | 0.2998 | 6.3653 | 0.3538 | 10.1749 |

**Table 22. % LC for various compositions of Cpd A.**

| **Time, week** | 0 | 1 | 1 | 2 | 2 |
|---|---|---|---|---|---|
| **RH** | | 11 % | 75 % | 11 % | 75 % |
| **Composition** | | | | | |
| Cpd **B** alone | 102.973 | 98.824 | 102.663 | 100.211 | 101.800 |
| Cpd **B** with with SMCC HD 90 | 101.294 | 98.919 | 94.656 | 99.785 | 97.355 |
| Cpd **B** with Avicel DG | 100.686 | 98.831 | 98.264 | 98.061 | 98.591 |
| Cpd **B** with Pearlitol 200 | 101.069 | 98.159 | 106.870 | 98.744 | 99.178 |
| Cpd **B** with Lactose anhydrous DT | 95.722 | 98.490 | 100.079 | 99.150 | 100.503 |
| Cpd **B** with Starch 1500 | 96.006 | 97.788 | 97.792 | 97.924 | 96.014 |
| Cpd **B** with Citric Acid | 102.767 | 102.104 | 82.265 | 102.603 | 67.003 |
| Cpd **B** with Fumaric acid | 97.236 | 101.115 | 100.882 | 100.395 | 101.072 |
| Cpd **B** with Tartaric Acid | 101.515 | 90.522 | 74.378 | 101.954 | 57.898 |
| Cpd **B** with Methocel E3 Premium LV | 97.985 | 100.989 | 87.710 | 100.269 | 91.767 |
| Cpd **B** with Povidone K30 | 100.637 | 93.607 | 93.630 | 99.547 | 94.779 |
| Cpd **B** with Croscarmellose sodium | 5.610 | 6.356 | 5.725 | 6.623 | 9.130 |
| Cpd **B** with Crospovidone | 101.128 | 98.723 | 101.047 | 100.487 | 97.175 |
| Cpd **B** with sodium starch glycolate | 56.110 | 56.344 | 48.821 | 55.370 | 37.815 |
| Cpd **B** with Colloidal Silicon Dioxide | 103.578 | 99.804 | 99.734 | 103.690 | 101.215 |
| Cpd **B** with Talc | 99.610 | 96.852 | 98.445 | 100.861 | 100.911 |
| Cpd **B** with Magnesium Stearate | 98.898 | 95.884 | 99.056 | 101.717 | 97.991 |
| Cpd **B** with Sodium stearyl fumarate | 103.035 | 101.988 | 101.166 | 101.897 | 99.324 |
| Cpd **B** with Stearic Acid 50 Vegetable grade | 104.835 | 102.700 | 102.644 | 103.109 | 101.309 |
| Cpd **B** with Opadry AMB II Blue | 103.238 | 102.957 | 91.651 | 100.040 | 87.089 |

### Example 2. Formulation studies.

The excipient compatibility study evaluated individual excipients as a binary mixture with Compound **B.** The second phase of development used the data from the excipient compatibility to combine the most stable excipients with three levels of stabilizers to evaluate the synergistic impact of these on the total RS for Compound **B.** Fumaric acid, citric acid, and tartaric acid were evaluated as stabilizers in blends simulated to deliver Compound **B** at 5 mg / dose and Compound **B** at 50 mg / dose to bracket the data between the lowest and highest dose. Each stabilizer was evaluated at a 1x and 5x ratio with Compound **B** at 5 mg dose, and at a 0.1x ratio with Compound **B** at 50 mg dose. A control formulation using the same excipients without the stabilizer was also set-up to evaluate the efficacy of the stabilizer against the excipients.

For each blend formulation listed in Table **23,** the required quantities of excipients and the API were individually dispensed by weight and screened through a 20-mesh sieve. The screened material was transferred to an appropriate size glass vial and blended manually for 5 minutes. Blend preparation included the addition of 3 glass beads and vortexing the closed vial to mix the ingredients for 30 - 60 seconds to ensure adequate mixing. Each blend formulation was prepared in 5 g batch size filled in 5 vials each. The vials of each blend were stored at both 50°C / 11% RH and 50°C / 75% RH conditions and sampled at t=0, 1 week, and 2 weeks. The blend was tested at each time point for assay and RS.

**Table 23. Formulations used for stability evaluation.**

| Ingredients | 5 mg blend, 5x stabilizer | | 5 mg blend, 1x stabilizer | | 50 mg blend, 0.1x stabilizer | | 5 mg blend, No stabilizer (Control) | |
|---|---|---|---|---|---|---|---|---|
| | % w/w | mg/unit | % w/w | mg/unit | % w/w | mg/unit | % w/w | mg/unit |
| Cpd **B¹** | 8.30 | 8.30 | 8.30 | 8.30 | 55.30 | 83.00 | 8.30 | 8.30 |
| Fumaric acid or Tartaric acid or Citric acid | 25.00 | 25.00 | 5.00 | 5.00 | 3.30 | 5.00 | 0.00 | 0.00 |
| Lactose anhydrous DT | 46.20 | 46.20 | 66.20 | 66.20 | 20.90 | 31.35 | 71.20 | 71.20 |
| Avicel DG | 15.00 | 15.00 | 15.00 | 15.00 | 15.00 | 22.50 | 15.00 | 15.00 |
| Polyplasdone XL | 5.00 | 5.00 | 5.00 | 5.00 | 5.00 | 7.50 | 5.00 | 5.00 |
| Magnesium stearate | 0.50 | 0.50 | 0.50 | 0.50 | 0.50 | 0.75 | 0.50 | 0.50 |
| Total | 100.00 | 100.00 | 100.00 | 100.00 | 100.00 | 150.00 | 100.00 | 100.00 |

| | | | | | | | | |
|---|---|---|---|---|---|---|---|---|
| ¹Cpd **B** base to salt conversion factor: 1.70 Potency (%) = (100 - KF - S) × P / 100 × MW Ratio Freebase / Bis-Salt KF = Water content by Karl Fischer = 1.6409% (C1780-142) S = Total residual solvents = 0.4090 (obtained from CofA) P = % Purity = 100.0% (obtained from CofA) MW = (Molecular weight of free base = 519.63) / (Molecular weight of Bis-Salt = 864.02) Potency (%) = (100-1.6409-0.4090) × 100.0 / 100 × 519.63 / 864.02 = 58.9081% Correction factor calculation Correction factor = 100 / Potency = 100 / 58.9081 = 1.70 | | | | | | | | |

### Example 3. Blends with fumaric acid.

Blends containing a 1:1 (1x), 1:5 (5x), and 10:1 (0.1x) ratio of Compound **B** base to fumaric acid presented an increase in **RS-1** in the range of ~0.05 % at t = 0 up to ~0.15% at t = 2 weeks and total RS from 0.05 % at t = 0 up to 0.3 % at t = 2 week at each stability condition (Table **24** - Table **26).** The total RS values for these blends were relatively lower when compared to the formulation that was manufactured without any acid stabilizer (Table **27).** It was noted that all blends containing fumaric acid responded similar in terms of degradation, and there was an unexpected trend in the data such that the samples stored at 50°C / 75 % RH had a relatively lower level of degradants as compared to samples stored at 50°C / 11 % RH.

The following related substances (RS) have been identified:
**RS-1**
**RS-2**
**RS-3**

**Table 24. Stability data with fumaric acid at 5X**

| **Time, week** | 0 | 1 | 1 | 2 | 2 |
|---|---|---|---|---|---|
| **Temp, °C** | -- | 50°C | 50°C | 50 °C | 50°C |
| **RH** | -- | 11% | 75% | 11% | 75% |
| **Cpd B % Recovery** | 103.48 | 102.45 | 85.38 | 102.153 | 107.683 |

| **Name / RRT** | | | | | |
|---|---|---|---|---|---|
| RS-1 | 0.062 | 0.073 | 0.054 | 0.077 | 0.067 |
| RS-2 | -- | -- | -- | 0.052 | 0.062 |
| 1.081 | -- | -- | -- | 0.121 | -- |
| 1.104 | -- | 0.05 | -- | -- | -- |
| **Total** | 0.0623 | 0.12 | 0.05 | 0.2496 | 0.1287 |

**Table 25. Stability data with fumaric acid at 1X**

| **Time, week** | 0 | 1 | 1 | 2 | 2 |
|---|---|---|---|---|---|
| **Temp, °C** | -- | 50°C | 50°C | 50 °C | 50°C |
| **RH** | -- | 11% | 75% | 11% | 75% |
| **Cpd B % Recovery** | 106.265 | 106.33 | 89.84 | 105.077 | 97.892 |

| **Name / RRT** | | | | | |
|---|---|---|---|---|---|
| RS-1 | 0.063 | 0.099 | 0.065 | 0.101 | 0.084 |
| RS-2 | -- | 0.056 | -- | 0.071 | 0.062 |
| 1.082 | -- | -- | -- | -- | 0.091 |
| 1.129 | -- | -- | -- | 0.061 | -- |
| **Total** | 0.0537 | 0.14 | 0.074 | 0.2551 | 0.1498 |

**Table 26. Stability data with fumaric acid at 0.1X**

| **Time, week** | 0 | 1 | 1 | 2 | 2 |
|---|---|---|---|---|---|
| **Temp, °C** | -- | 50°C | 50°C | 50 °C | 50°C |
| **RH** | -- | 11% | 75% | 11% | 75% |
| **Cpd B % Recovery** | 90.363 | 99.47 | 87.86 | 105.234 | 96.598 |

| **Name / RRT** | | | | | |
|---|---|---|---|---|---|
| RS-1 | 0.054 | 0.093 | 0.074 | 0.121 | 0.092 |
| RS-2 | -- | 0.051 | -- | 0.071 | -- |
| 1.038 | -- | -- | -- | 0.063 | -- |
| 1.082 | -- | -- | -- | -- | 0.058 |
| **Total** | 0.0537 | 0.14 | 0.074 | 0.2551 | 0.1498 |

**Table 27. Stability data without stabilizer**

| **Time, week** | 0 | 1 | 1 | 2 | 2 |
|---|---|---|---|---|---|
| **Temp, °C** | -- | 50°C | 50°C | 50 °C | 50°C |
| **RH** | -- | 11% | 75% | 11% | 75% |
| **Cpd B % Recovery** | 101.362 | 107.76 | 103.26 | 99.796 | 98.595 |

| **Name / RRT** | | | | | |
|---|---|---|---|---|---|
| RS-1 | 0.058 | 0.12 | 0.126 | 0.145 | 0.163 |
| RS-2 | -- | 0.056 | -- | 0.069 | 0.059 |
| RS-3 | -- | -- | -- | 0.084 | -- |
| 0.544 | -- | -- | -- | -- | 0.053 |
| 1.082 | -- | -- | -- | -- | 0.075 |
| **Total** | 0.0584 | 0.18 | 0.13 | 0.2988 | 0.3499 |

### Example 4. Blends with citric acid.

Blends containing a 1:1 (1x), 1:5 (5x), and 10:1 (0.1x) ratio of Compound **B** base to citric acid presented an increase in **RS-1** in the range of ~0.05 % at t = 0 up to ~0.17 % at t = 2 weeks and total RS from 0.05 % at t = 0 up to 5.2 % at t = 2 week at each stability condition (Table **28** - Table **30).**

**Table 28. Stability data with citric acid at 5X**

| **Time, week** | 0 | 1 | 1 | 2 | 2 |
|---|---|---|---|---|---|
| **Temp, °C** | -- | 50°C | 50°C | 50 °C | 50 °C |
| **RH** | -- | 11% | 75% | 11% | 75% |
| **Cpd B % Recovery** | 98.196 | 110.71 | 110.7 | 113.789 | 103.237 |

| **Name / RRT** | | | | | |
|---|---|---|---|---|---|
| RS-1 | 0.058 | 0.083 | 0.13 | 0.106 | 0.159 |
| RS-2 | -- | 0.056 | 0.053 | 0.067 | -- |
| 1.037 | -- | -- | -- | 0.07 | -- |
| 1.082 | -- | -- | -- | -- | 0.065 |
| 1.16 | -- | -- | 1.587 | -- | 0.433 |
| 1.193 | -- | -- | -- | -- | 4.576 |
| **Total** | 0.0579 | 0.14 | 1.77 | 0.2426 | 5.2331 |

**Table 29. Stability data with citric acid at 1X**

| **Time, week** | 0 | 1 | 1 | 2 | 2 |
|---|---|---|---|---|---|
| **Temp, °C** | -- | 50°C | 50°C | 50 °C | 50°C |
| **RH** | -- | 11% | 75% | 11% | 75% |
| **Cpd B % Recovery** | 102.899 | 98.6 | 117.5 | 104.335 | 106.174 |

| **Name / RRT** | | | | | |
|---|---|---|---|---|---|
| RS-1 | 0.061 | 0.074 | 0.098 | 0.097 | 0.173 |
| RS-2 | 0.051 | 0.053 | 0.066 | 0.059 | -- |
| 1.037 | -- | -- | -- | 0.082 | -- |
| 1.082 | -- | -- | -- | -- | 0.073 |
| 1.16 | -- | -- | -- | -- | 0.113 |
| 1.193 | -- | -- | -- | -- | 1.478 |
| **Total** | 0.1125 | 0.13 | 0.16 | 0.2383 | 1.8382 |

**Table 30. Stability data with citric acid at 0.1X**

| | | | | | |
|---|---|---|---|---|---|
| **Time, week** | 0 | 1 | 1 | 2 | 2 |
| **Temp, °C** | -- | 50°C | 50°C | 50 °C | 50°C |
| **RH** | -- | 11% | 75% | 11% | 75% |
| **Cpd B % Recovery** | 104.152 | 104.01 | 99.74 | 104.591 | 102.659 |

| **Name / RRT** | | | | | |
|---|---|---|---|---|---|
| RS-1 | 0.064 | 0.091 | 0.075 | 0.12 | 0.096 |
| RS-2 | -- | 0.054 | -- | 0.068 | 0.054 |
| 1.038 | -- | -- | -- | 0.059 | -- |
| 1.082 | -- | -- | -- | -- | 0.059 |
| 1.193 | -- | -- | -- | -- | 0.18 |
| **Total** | 0.064 | 0.15 | 0.075 | 0.2463 | 0.3886 |

### Example 5. Blends with tartaric acid.

Blends containing a 1:1 (1x), 1:5 (5x), and 10:1 (0.1x) ratio of Compound **B** base to tartaric acid presented an increase in **RS-1** in the range of ~0.05 % at t = 0 up to ~0.11 % at t = 2 weeks and total RS from 0.05 % at t = 0 up to 2.5 % at t = 2 week at each stability condition (Table **31 -** Table **33).**

**Table 31. Stability data with tartaric acid at 5X**

| | | | | | |
|---|---|---|---|---|---|
| **Time, week** | 0 | 1 | 1 | 2 | 2 |
| **Temp, °C** | -- | 50 °C | 50 °C | 50 °C | 50 °C |
| **RH** | -- | 11% | 75% | 11% | 75% |
| **Cpd B % Recovery** | 70.451 | 98.47 | 82.03 | 99.15 | 99.759 |

| **Name / RRT** | | | | | |
|---|---|---|---|---|---|
| RS-1 | -- | 0.064 | 0.054 | 0.083 | 0.075 |
| RS-2 | -- | 0.051 | -- | 0.052 | -- |
| 1.082 | -- | -- | -- | 0.086 | -- |
| 1.113 | -- | -- | -- | -- | 0.347 |
| 1.16 | -- | -- | -- | -- | 0.199 |
| 1.1195 | -- | -- | -- | -- | 1.957 |
| **Total** | -- | 0.12 | 0.21 | 0.2215 | 2.5785 |

**Table 32. Stability data with tartaric acid at 1X**

| | | | | | |
|---|---|---|---|---|---|
| **Time, week** | 0 | 1 | 1 | 2 | 2 |
| **Temp, °C** | -- | 50 °C | 50 °C | 50 °C | 50 °C |
| **RH** | -- | 11% | 75% | 11% | 75% |
| **Cpd B % Recovery** | 100.305 | 98.08 | 91.57 | 113.18 | 102.463 |

| **Name / RRT** | | | | | |
|---|---|---|---|---|---|
| RS-1 | 0.061 | 0.073 | 0.062 | 0.095 | 0.092 |
| RS-2 | -- | -- | 0.057 | 0.076 | -- |
| RS-3 | -- | 0.06 | -- | -- | -- |
| 1.038 | -- | -- | -- | 0.074 | -- |
| 1.086 | -- | -- | 0.077 | -- | -- |
| 1.128 | -- | -- | -- | 0.053 | 0.12 |
| 1.153 | -- | -- | 0.245 | -- | 0.128 |
| 1.1195 | -- | -- | -- | -- | 1.201 |
| **Total** | 0.0606 | 0.13 | 0.44 | 0.2976 | 1.5411 |

**Table 33. Stability data with tartaric acid at 0.1X**

| | | | | | |
|---|---|---|---|---|---|
| **Time, week** | 0 | 1 | 1 | 2 | 2 |
| **Temp, °C** | -- | 50 °C | 50 °C | 50 °C | 50 °C |
| **RH** | -- | 11% | 75% | 11% | 75% |
| **Cpd B % Recovery** | 101.081 | 100.85 | 94.27 | 103.508 | 96.957 |

| **Name / RRT** | | | | | |
|---|---|---|---|---|---|
| RS-1 | 0.061 | 0.079 | 0.473 | 0.111 | 0.11 |
| RS-2 | -- | -- | -- | -- | 0.052 |
| RS-3 | -- | -- | 0.051 | | |
| 0.527 | -- | -- | 0.101 | -- | -- |
| 1.068 | -- | -- | 0.432 | -- | -- |
| 1.091 | -- | -- | 0.22 | 0.089 | 0.067 |
| 1.11 | -- | -- | 0.24 | -- | -- |
| 1.158 | -- | -- | 0.602 | -- | -- |
| 1.125 | -- | -- | 0.056 | -- | -- |
| **Total** | 0.0613 | 0.08 | 2.18 | 0.2002 | 0.2288 |

### Example 6. Manufacturing process and dosage form.

The results from blend evaluation study were used to design the next set of experiments for manufacturing process selection and dosage form selection. A tablet and capsule delivery system were evaluated using a direct blend and a wet-granulation approach for the dose range of 5 mg - 50 mg. The qualitative composition of the blend was maintained similar to that evaluated in the blend study.

In addition to this, a control formulation was designed without fumaric acid, and substituting PolyplasdoneTM XL with Starch 1500^{®}.

A series of 13 experiments were designed to evaluate the impact of manufacturing process and dosage form. Experiments 1 - 3 were designed as a single common direct blend split three ways to formulate the 5 mg and 10 mg doses as dose proportional formulations. The first portion was used to manufacture Compound **B** tablets, 5 mg, second portion was used to manufacture Compound **B** tablets, 10 mg, and third portion to manufacture Compound **B** capsules, 5 mg. Similarly, experiments 4 - 6 were designed as a single common wet-granulation blend split three ways. The first portion was used to manufacture Compound **B** tablets, 5 mg, second portion was used to manufacture Compound **B** tablets, 10 mg, and third portion to manufacture Compound **B** capsules, 5 mg. The 35 mg and 50 mg tablets and capsules were designed to be dose similar formulations at a blend weight of 200 mg / dose. Tablets were compressed by individually weighing blend for each tablet on a balance, manually filling the die, and compression using a single punch on a rotary press. Encapsulation was performed manually on an analytical balance. The dosage and manufacturing process of for each of 13 total experiments are listed below.

| Expt. No. | Form | Dose | Blend | Fumaric acid? |
|---|---|---|---|---|
| 1 | Tablets | 5 mg | Direct Blend | Y |
| 2 | Tablets | 10 mg | Direct Blend | Y |
| 3 | Capsules | 5 mg | Direct Blend | Y |
| 4 | Tablets | 5 mg | Wet Granulation | Y |
| 5 | Tablets | 10 mg | Wet Granulation | Y |
| 6 | Capsules | 5 mg | Wet Granulation | Y |
| 7 | Tablets | 35 mg | Direct Blend | Y |
| 8 | Tablets | 35 mg | Wet Granulation | Y |
| 9 | Capsules | 50 mg | Direct Blend | Y |
| 10 | Capsules | 50 mg | Wet Granulation | Y |
| 11 | Tablets | 5 mg | Direct Blend | N |
| 12 | Tablets | 10 mg | Direct Blend | N |
| 13 | Tablets | 35 mg | Direct Blend | N |

**Table 34. Direct blend formulations.**

| Expt # | #1: Tablets 5 mg | | | #7: Tablets 35 mg | | #9: 50 mg capsules | |
|---|---|---|---|---|---|---|---|
| | #2: Tablets 10 mg | | | | | | |
| | #3: 5 mg capsules | | | | | | |
| Ingredient | 5mg / 10 mg common blend | 5 mg strength | 10 mg strength | 35 mg strength | | 50 mg strength | |
| | % w/w | mg/unit | | % w/w | mg/unit | % w/w | mg/unit |
| Compound **B** free base | 5.00 | 5.00 (8.35)¹ | 10.00 (16.70)¹ | 17.50 | 35.00 (58.45)¹ | 25.00 | 50.00 (83.50)¹ |
| Fumaric acid | 5.00 | 5.00 | 10.00 | 17.50 | 35.00 | 25.00 | 50.00 |
| Lactose anhydrous | 69.50 | 69.50 (66.15)¹ | 139.00 (132.30)¹ | 44.50 | 89.00 (65.55)¹ | 29.50 | 59.00 (25.50)¹ |
| Avicel DG | 15.00 | 15.00 | 30.00 | 15.00 | 300 | 15.00 | 30.00 |
| Crospovidone | 5.00 | 5.00 | 10.00 | 5.00 | 100 | 5.00 | 10.00 |
| Magnesium stearate | 0.50 | 0.50 | 1.00 | 0.50 | 1.00 | 0.50 | 1.00 |
| Total | 100.00 | 100.00 | 200.00 | 100.00 | 200.00 | 100.00 | 200.00 |

| | | | | | | | |
|---|---|---|---|---|---|---|---|
| *Potency calculation Potency (%) = (100 - KF - S) × P / 100 × MW Ratio Freebase / Bis-Salt KF = Water content by Karl Fischer = 0.3 (obtained from CofA) S = Total residual solvents = 0.1234 (obtained from CofA) P = % Purity = 99.95% (obtained from CofA) MW = (Molecular weight of free base = 519.63) / (Molecular weight of Bis-Salt = 864.02) Potency (%) = (100-0.3-0.1234) × 99.95 / 100 × 519.63 / 864.02 = 59.8564% Correction factor calculation Correction factor = 100 / Potency = 100 / 59.8564 = 1.67 | | | | | | | |

**Table 35. Wet-granulation formulations.**

| Expt # | #4: Tablets 5 mg | | | #8: Tablets 35 mg | | #10: 50 mg capsules | |
|---|---|---|---|---|---|---|---|
| | #5: Tablets 10 mg | | | | | | |
| | #6: 5 mg capsules | | | | | | |
| Ingredient | 5mg / 10mg common blend | 5 mg strength | 10 mg strength | 35 mg strength | | 50 mg strength | |
| | % w/w | mg/unit | | % w/w | mg/unit | % w/w | mg/unit |
| Intragranular Ingredients | | | | | | | |
| Compound **B** free base | 5.00 | 5.00 (8.35)¹ | 10.00 (16.70)¹ | 17.50 | 35.00 (58.45)¹ | 25.00 | 50.00 (83.50)¹ |
| Fumaric acid | 5.00 | 5.00 | 10.00 | 17.50 | 35.00 | 25.00 | 50.00 |
| Lactose anhydrous | 49.50 | 49.50 (46.15)¹ | 99.00 (92.30)¹ | 24.50 | 49.00 (25.53)¹ | 24.50 | 49.00 (15.50)¹ |
| Avicel DG | 15.00 | 15.00 | 30.00 | 15.00 | 30.00 | 15.00 | 30.00 |

| Extragranular Ingredients | | | | | | | |
|---|---|---|---|---|---|---|---|
| Lactose anhydrous | 20.00 | 20.00 | 40.00 | 20.00 | 40.00 | 5.00 | 10.00 |
| Crospovidone | 5.00 | 5.00 | 10.00 | 5.00 | 10.00 | 5.00 | 10.00 |
| Magnesium stearate | 0.50 | 0.50 | 1.00 | 0.50 | 1.00 | 0.50 | 1.00 |
| Total | 100.00 | 100.00 | 200.00 | 100.00 | 200.00 | 100.00 | 200.00 |

| | | | | | | | |
|---|---|---|---|---|---|---|---|
| *Potency calculation Potency (%) = (100 - KF - S) × P / 100 × MW Ratio Freebase / Bis-Salt KF = Water content by Karl Fischer = 0.3 (obtained from CofA) S = Total residual solvents = 0.1234 (obtained from CofA) P = % Purity = 99.95% (obtained from CofA) MW = (Molecular weight of free base = 519.63) / (Molecular weight of Bis-Salt = 864.02) Potency (%) = (100-0.3-0.1234) × 99.95 / 100 × 519.63 / 864.02 = 59.8564% Correction factor calculation Correction factor = 100 / Potency = 100 / 59.8564 = 1.67 | | | | | | | |

**Table 36. Control formulations.**

| Expt # | #11: Tablets 5 mg | | | #13: Tablets 35 mg | |
|---|---|---|---|---|---|
| | #12: Tablets 10 mg | | | | |
| Ingredient | % w/w | 5 mg strength | 10 mg strength | % w/w | 35 mg strength |
| | | mg/unit | | | |
| Compound **B** free base | 5.00 | 5.00 (8.35)¹ | 10.00 (16.70)¹ | 17.50 | 35.00 (58.45)¹ |
| Lactose anhydrous | 74.50 | 74.50 (71.15)¹ | 149.00 (142.30)¹ | 62.00 | 124.00 (100.55)¹ |
| Avicel DG | 15.00 | 15.00 | 30.00 | 15.00 | 30.00 |
| Starch 1500 | 5.00 | 5.00 | 10.00 | 5.00 | 10.00 |
| Magnesium stearate | 0.50 | 0.50 | 1.00 | 0.50 | 1.00 |
| Total | 100.00 | 100.00 | 200.00 | 100.00 | 200.00 |

10 units of finished product (tablets or capsules) from each or the 13 batches were packaged into 30 cc bottles, induction sealed, and torqued with 28 mm caps. A total of 7 bottles were packaged for each of the batches and evaluated for stability in two accelerated conditions: 50°C / 75 % RH, 50°C / 11 % RH. The samples were evaluated at t = 0, 1 week, 2 weeks, and 5 weeks time points for assay / RS.

Bottles used: 30 cc wide mouth pharmaceutical round white bottle

Drug plastics and closures Inc Item #0030GAX101

Caps used: 28 mm SecuRx RbTx White FS M1 w / .035 Pulp Prt 'SFYP' wht

Drug plastics and closures Inc Item #28CRG11101

The results of this study were very surprising and very clear. Tablet formulations manufactured via direct blend or wet granulation, with or without fumaric acid, all were observed to be extremely unstable as compared to the data observed in excipient compatibility and blend stability studies.

**Direct Blend** Compound **B** common blend was manufactured via direct blend and used to make tablets (5 mg and 10 mg) and capsules (5 mg). The total RS for 5 mg tablets increased from 0.12 % at t = 0 to 0.92 % for the sample stored at 50°C / 75 % RH up to t = 5 weeks (Table **37).** Similarly, the total RS for 10 mg tablets increased from 0.11 % at t = 0 to 0.71 % for the sample stored at 50°C / 75 % RH up to t = 5 weeks (Table **38).** Capsules (5 mg) were relatively stable as compared to the tablets. The total RS for capsules was 0.11% at t = 0 and increased up to 0.17 % after 5 weeks of storage at 50°C / 75% RH (Table **39).**

Both tablets (35 mg) and capsules (50 mg) were also manufactured using the direct blend manufacturing process. The total RS for tablets (35 mg) increased from 0.12 % at t = 0 to 0.44 % at t = 2 weeks 50°C / 75% RH and 0.39 % at t = 5 weeks 50°C / 75% RH (Table **43).** The 50 mg capsules were relatively stable as compared to the 35 mg tablets. The total RS for capsules (50 mg) was 0.11 % at t = 0 increased to 0.17 % at t = 2 weeks 50°C / 75% RH and 0.12 % at 5 weeks 50°C / 75% RH (Table **45).**

**Wet granulation** A common blend of Compound **B** was manufactured via wet-granulation to make tablets (5 mg and 10 mg) and capsules (5 mg). The total RS for 5 mg tablets increased from 0.10 % at t = 0 to 1.76 % for the sample stored at 50°C / 75 % RH up to t = 5 weeks (Table **40).** The total RS for 10 mg tablets increased from 0.10 % at t = 0 to 1.51 % for the sample stored at 50°C / 75 % RH up to t = 5 weeks (Table **41).** The total RS for capsules (5 mg) increased from 0.10 % at t = 0 to 0.78 % at t = 5 weeks of storage at 50°C / 75% RH (Table **42).**

Both tablets (35 mg) and capsules (50 mg) were also manufactured following wet granulation. The total RS in tablets (35 mg) was observed to increase from 0.10 % at t = 0 to 0.64% after 5 weeks of storage at 50°C / 75% RH (Table **44).** The total RS in capsules increased from 0.11% at t = 0 to 0.26 % after 5 weeks of storage at 50°C / 75% RH (Table 46).

The data for wet-granulation trends similar to that observed with direct blend, Compound **B** capsules were relatively stable compared to the tablets manufactured via wet-granulation. However, the increase in total RS for capsules manufactured via wet-granulation was greater than that observed in capsules manufactured via direct blend.

**Control formulation** The three control formulations manufactured without fumaric acid and crospovidone, with added Starch 1500^{®} presented with the highest level of RS (Tables **47, 48,** and **49,** for 5 mg, 10 mg, and 35 mg tablets, respectively). These were not evaluated any further.

The tablets and capsules for a given manufacturing process were all generated from a common blend thus eliminating any bias in the interpretation of the results obtained. Overall, capsules of Compound **B** are significantly more stable compared to tablets of Compound **B.** Capsules manufactured via a direct blend manufacturing process present better stability as compared to capsules manufactured via wet-granulation. This was surprising as a compressed tablet is conventionally assumed to be more stable than a capsule due to the presence of moisture in the capsule shell. Our hypothesis is that the compression force used in generating a tablet impacts the crystalline structure of the drug substance such that it accelerates the degradation. The capsule formulation manufactured using the direct blend process also performed similar to the blend stability data generated in the section 3, further suggesting that the compression forces in manufacturing a tablet may have an impact to stability. HPMC capsules used in this formulation are designed to be suitable for moisture sensitive and hygroscopic blends, thus it is inferred that the moisture in these capsules is tightly bound and may not be available for hydrolysis.

**Table 37. Stability profile for 5 mg tablets manufactured via direct blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 110.0 | 107.17 | 107.1 | 112.4 | 110.3 | 103 | 103.6 |

| **Name / RRT** | | | | | | | |
|---|---|---|---|---|---|---|---|
| 0.173 | 8.34 | 8.46 | 8.66 | 8.91 | 8.6 | 7.81 | 8.36 |
| RS-1 | 0.12 | 0.27 | 0.3 | 0.34 | 0.36 | 0.36 | 0.5 |
| 0.400 | | | | | | | 0.28 |
| 0.428 | | | | | | | 0.06 |
| 0.501 | | | | | | | 0.03 |
| 0.680 | | | | | | | 0.04 |
| 1.028 | | 0.04 | 0.44 | 0.06 | 0.05 | 0.05 | 0.07 |
| RS-2 | | 0.04 | 0.03 | 0.03 | | | 0.03 |
| 1.050 | | | 0.04 | 0.05 | 0.05 | 0.04 | 0.04 |
| 1.069 | | | | | | | 0.03 |
| 1.140 | 0.04 | 0.04 | 0.04 | 0.04 | 0.04 | 0.03 | 0.03 |
| 2.031 | | | | | | | 0.03 |
| **Total** | 0.12 | 0.27 | 0.3 | 0.45 | 0.46 | 0.4 | 0.92 |

**Table 38. Stability profile for 10 mg tablets manufactured via direct blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 103.9 | 103.7 | 101.3 | 106.5 | 107.2 | 104.2 | 101.7 |

| **Name / RRT** | | | | | | | |
|---|---|---|---|---|---|---|---|
| 0.173 | 9.09 | 9.17 | 8.83 | 9.44 | 9.57 | 9.06 | 9.38 |
| RS-1 | 0.11 | 0.29 | 0.31 | 0.38 | 0.38 | 0.43 | 0.45 |
| 0.403 | | | | | | | 0.21 |
| 0.431 | | | | | | | 0.04 |
| 0.681 | | | | | | | 0.03 |
| 1.031 | | 0.043 | 0.04 | 0.05 | 0.05 | | 0.05 |
| RS-2 | | 0.035 | 0.03 | 0.03 | 0.03 | 0.04 | 0.03 |
| 1.045 | | | | | | 0.04 | |
| 1.049 | 0.04 | | 0.03 | 0.05 | 0.04 | 0.03 | |
| 1.140 | 0.04 | 0.04 | 0.04 | 0.04 | 0.04 | 0.03 | |
| **Total** | 0.11 | 0.29 | 0.31 | 0.48 | 0.43 | 0.48 | 0.71 |

**Table 39. Stability profile for 5 mg capsules manufactured via direct blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 102.9 | 99.5 | 102.6 | 98.6 | 103.2 | 99.3 | 97 |

| **Name / RRT** | | | | | | | |
|---|---|---|---|---|---|---|---|
| 0.173 | 8.34 | 8.9 | 9.35 | 8.58 | 9.11 | 8.55 | 8.6 |
| RS-1 | 0.11 | 0.11 | 0.11 | 0.12 | 0.12 | 0.12 | 0.12 |
| 1.028 | | 0.04 | 0.04 | 0.05 | 0.05 | | 0.05 |
| RS-2 | | 0.03 | 0.03 | 0.03 | 0.03 | 0.03 | 0.03 |
| 1.042 | | | 0.03 | 0.04 | 0.04 | 0.03 | 0.03 |
| 1.140 | 0.04 | 0.04 | 0.04 | 0.04 | 0.04 | 0.03 | 0.03 |
| **Total** | 0.11 | 0.11 | 0.11 | 0.16 | 0.17 | 0.12 | 0.17 |

**Table 40. Stability profile for 5 mg tablets manufactured via wet granulation blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 91.3 | 91.8 | 94 | 94.8 | 95.9 | 91.5 | 85.8 |

| **Name / RRT** | | | | | | | |
|---|---|---|---|---|---|---|---|
| 0.173 | 9.17 | 9.47 | 9.38 | 9.8 | 9.71 | 9.68 | 9.11 |
| RS-1 | 0.10 | 0.2 | 0.21 | 0.26 | 0.33 | 0.33 | 0.53 |
| 0.291 | | | | | | | 0.03 |
| 0.403 | | | 0.04 | | 0.23 | | 1.03 |
| 0.432 | | | | | | | 0.15 |
| 0.563 | | | | | | | 0.04 |
| 1.031 | | 0.04 | 0.04 | 0.04 | 0.04 | | 0.05 |
| 1.042 | | 0.03 | 0.03 | 0.04 | 0.04 | 0.03 | |
| RS-2 | | 0.03 | 0.03 | 0.03 | | 0.04 | |
| 1.139 | 0.03 | 0.03 | 0.03 | | 0.03 | | |
| 2.022 | | | | 0.07 | 0.05 | 0.04 | 0.04 |
| **Total** | 0.10 | 0.2 | 0.21 | 0.33 | 0.61 | 0.33 | 1.76 |

**Table 41. Stability profile for 10 mg tablets manufactured via wet granulation blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 89.8 | 89.8 | 89.1 | 91.9 | 90.8 | 91.7 | 88.1 |

| **Name** / **RRT** | | | | | | | |
|---|---|---|---|---|---|---|---|
| 0.173 | 9.24 | 9.1 | 9.16 | 9.35 | 9.48 | 9.74 | 9.32 |
| RS-1 | 0.10 | 0.19 | 0.19 | 0.24 | 0.27 | 0.32 | 0.44 |
| 0.404 | | | | | 0.14 | 0.04 | 0.87 |
| 0.433 | | | | | | | 0.1 |
| 0.564 | | | | | | | 0.03 |
| 1.031 | | 0.04 | 0.04 | 0.04 | 0.04 | | 0.05 |
| 1.042 | | 0.03 | 0.03 | 0.04 | 0.03 | 0.03 | |
| RS-2 | | 0.03 | 0.03 | 0.02 | 0.03 | 0.03 | |
| 1.139 | 0.03 | 0.03 | 0.03 | 0.03 | 0.03 | | |
| 2.032 | | 0.05 | 0.1 | 0.08 | 0.07 | 0.11 | 0.06 |
| 2.022 | | | | | | | 0.03 |
| **Total** | 0.10 | 0.19 | 0.3 | 0.32 | 0.48 | 0.43 | 1.51 |

**Table 42. Stability profile for 5 mg capsules manufactured via wet granulation blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 92.1 | 91.1 | 90.8 | 93.4 | 95.6 | 91.2 | 93 |

| **Name / RRT** | | | | | | | |
|---|---|---|---|---|---|---|---|
| 0.174 | 8.83 | 8.94 | 8.88 | 8.74 | 9.3 | 8.32 | 9.03 |
| RS-1 | 0.10 | 0.15 | 0.15 | 0.19 | 0.2 | 0.07 | 0.25 |
| 0.396 | | | 0.04 | 0.07 | 0.16 | | 0.42 |
| 0.424 | | | | | | 0.35 | 0.03 |
| 1.028 | | 0.04 | 0.04 | 0.04 | 0.04 | | 0.05 |
| 1.042 | | | 0.03 | 0.04 | 0.04 | 0.03 | |
| RS-2 | | 0.03 | 0.03 | 0.03 | 0.03 | 0.03 | 0.02 |
| 1.139 | 0.03 | 0.03 | 0.03 | 0.03 | 0.03 | | |
| 2.031 | | | 0.05 | 0.075 | | 0.04 | 0.06 |
| **Total** | 0.10 | 0.15 | 0.15 | 0.33 | 0.36 | 0.42 | 0.78 |

**Table 43. Stability profile for 35 mg tablets manufactured via direct blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 111.3 | 108.3 | 109.1 | 112.1 | 112.5 | 111.5 | 107.4 |

| **Name / RRT** | | | | | | | |
|---|---|---|---|---|---|---|---|
| 0.173 | 9.26 | 9.1 | 9.3 | 9.4 | 9.47 | 9.44 | 9.11 |
| RS-1 | 0.12 | 0.25 | 0.28 | 0.34 | 0.32 | 0.42 | 0.32 |
| 0.405 | | | | | | | 0.07 |
| 1.027 | | 0.04 | 0.04 | 0.05 | 0.06 | | 0.04 |
| 1.049 | 0.04 | 0.04 | 0.04 | 0.05 | 0.05 | 0.04 | |
| 1.052 | | | | | | 0.04 | |
| RS-2 | | 0.04 | 0.03 | 0.03 | 0.04 | 0.04 | |
| 1.140 | 0.05 | 0.04 | 0.04 | 0.04 | 0.04 | 0.03 | 0.03 |
| **Total** | 0.12 | 0.25 | 0.28 | 0.44 | 0.44 | 0.42 | 0.39 |

**Table 44. Stability profile for 35 mg tablets manufactured via wet granulation blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 91.3 | 89 | 87.9 | 87 | 90.9 | 86.9 | 86.6 |

| **Name / RRT** | | | | | | | |
|---|---|---|---|---|---|---|---|
| 0.173 | 9.11 | 9.08 | 8.96 | 8.89 | 9.13 | 8.99 | 8.91 |
| RS-1 | 0.10 | 0.15 | 0.16 | 0.17 | 0.19 | 0.2 | 0.25 |
| 0.403 | | | | | | | 0.29 |
| 1.028 | | 0.04 | 0.04 | 0.04 | 0.04 | | 0.03 |
| 1.042 | | 0.03 | 0.03 | 0.04 | 0.03 | 0.03 | |
| RS-2 | | 0.03 | 0.03 | 0.03 | 0.02 | 0.03 | |
| 1.139 | 0.03 | 0.03 | 0.03 | 0.03 | 0.03 | | |
| 1.071 | | | | | | | 0.1 |
| **Total** | 0.10 | 0.15 | 0.16 | 0.17 | 0.19 | 0.2 | 0.64 |

**Table 45. Stability profile for 50 mg capsules manufactured via direct blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 94.1 | 98.1 | 100.7 | 100 | 104.5 | 101.2 | 105.7 |

| **Name / RRT** | | | | | | | |
|---|---|---|---|---|---|---|---|
| 0.173 | 8.47 | 9.03 | 9.18 | 9.17 | 9.44 | 9.21 | 9.66 |
| RS-1 | 0.10 | 0.11 | 0.12 | 0.12 | 0.12 | 0.12 | 0.12 |
| 1.028 | | 0.04 | 0.04 | 0.04 | 0.05 | | 0.05 |
| 1.042 | | 0.04 | 0.04 | 0.04 | 0.04 | 0.03 | 0.04 |
| RS-2 | | 0.03 | 0.03 | 0.03 | 0.03 | 0.03 | 0.03 |
| 1.139 | 0.04 | 0.04 | 0.04 | 0.04 | 0.04 | | 0.03 |
| **Total** | 0.10 | 0.11 | 0.12 | 0.12 | 0.17 | 0.12 | 0.12 |

**Table 46. Stability profile for 50 mg capsules manufactured via wet granulation blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 92.3 | 96.2 | 96.4 | 97.4 | 96.3 | 95.5 | 95.4 |

| **Name / RRT** | | | | | | | |
|---|---|---|---|---|---|---|---|
| 0.173 | 9.13 | 9.69 | 9.7 | 9.72 | 9.74 | 9.62 | 9.58 |
| RS-1 | 0.10 | 0.12 | 0.12 | 0.12 | 0.12 | 0.12 | 0.14 |
| 0.403 | | | | | | | 0.12 |
| 1.029 | | 0.04 | 0.04 | 0.04 | 0.05 | | 0.04 |
| 1.042 | | 0.04 | 0.03 | 0.04 | 0.04 | 0.04 | |
| RS-2 | | 0.03 | 0.03 | 0.03 | 0.03 | 0.03 | 0.02 |
| 1.139 | 0.03 | 0.04 | 0.04 | 0.04 | 0.03 | | |
| **Total** | 0.10 | 0.12 | 0.12 | 0.12 | 0.17 | 0.12 | 0.26 |

**Table 47. Stability profile for 5 mg tablets, control formulation manufactured via direct blend.**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 99.7 | 97.8 | 99.9 | 100.2 | 98.9 | 97 | 94.5 |

| **Name / RRT** | | | | | | | |
|---|---|---|---|---|---|---|---|
| 0.170 | 0.08 | 0.14 | 0.13 | 0.13 | 0.11 | 0.13 | 0.03 |
| RS-1 | 0.11 | 0.44 | 0.52 | 0.54 | 0.65 | 0.65 | 1 |
| 0.396 | | | 0.06 | | 0.21 | | 0.78 |
| 0.434 | | | | | | | 0.12 |
| 0.477 | | | | | | | 0.04 |
| 0.534 | | | | | | | 0.04 |
| 0.683 | | | | | 0.04 | | 0.12 |
| 0.842 | | | | | | | 0.09 |
| 1.029 | | 0.04 | 0.04 | 0.04 | 0.05 | | 0.05 |
| 1.049 | 0.03 | 0.03 | 0.03 | 0.04 | | 0.03 | |
| RS-2 | | 0.03 | 0.03 | 0.03 | 0.03 | 0.04 | |
| 1.108 | | | | | 0.03 | 0.03 | 0.04 |
| 1.140 | 0.04 | 0.04 | 0.04 | 0.04 | 0.04 | 0.03 | 0.03 |
| 1.965 | | | | | | | 0.09 |
| **Total** | 0.11 | 0.44 | 0.6 | 0.54 | 0.92 | 0.65 | 2.36 |

**Table 48. Stability profile for 10 mg tablets, control formulation manufactured via direct blend**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 99.1 | 98.6 | 97.6 | 102 | 99.2 | 95.4 | 93.7 |

| **Name / RRT** | | | | | | | |
|---|---|---|---|---|---|---|---|
| 0.173 | 0.10 | 0.13 | 0.13 | 0.13 | 0.11 | 0.14 | 0.03 |
| RS-1 | 0.11 | 0.44 | 0.45 | 0.58 | 0.66 | 0.66 | 0.77 |
| 0.406 | | | | | 0.22 | | 0.65 |
| 0.434 | | | | | | | 0.09 |
| 0.534 | | | | | | | 0.03 |
| 0.683 | | | | | 0.04 | | 0.1 |
| 0.809 | | | | | | | 0.03 |
| 0.842 | | | | | | | 0.08 |
| 1.031 | | 0.04 | 0.04 | 0.05 | 0.04 | | 0.06 |
| 1.112 | | | | | | | 0.04 |
| 1.049 | 0.03 | 0.03 | 0.03 | 0.04 | | | |
| RS-2 | | 0.03 | 0.03 | 0.03 | 0.03 | 0.04 | |
| 1.109 | | | | | 0.04 | 0.03 | |
| 1.140 | 0.04 | 0.04 | 0.04 | 0.04 | 0.04 | 0.03 | 0.03 |
| 1.968 | | | | | | | 0.07 |
| Total | 0.11a | 0.44 | 0.45 | 0.63 | 0.88 | 0.66 | 1.82 |

**Table 49. Stability profile for 35 mg tablets, control formulation manufactured via direct blend**

| **Time, week** | 0 | 1 | 1 | 2 | 2 | 5 | 5 |
|---|---|---|---|---|---|---|---|
| **Temp, °C** | | 50 °C | 50 °C | 50 °C | 50 °C | 50 °C | 50 °C |
| **RH** | | 11 % | 75 % | 11 % | 75 % | 11 % | 75 % |
| **% LC** | 99.8 | 102.1 | 102.6 | 104 | 98.2 | 99.2 | 97.4 |

| **Name / RRT** | | | | | | | |
|---|---|---|---|---|---|---|---|
| 0.173 | 0.10 | 0.1 | 0.12 | | 0.08 | 0.12 | 0.05 |
| RS-1 | 0.11 | 0.35 | 0.55 | 0.49 | 0.79 | 0.63 | 0.84 |
| 0.405 | | | | | 0.07 | | 0.74 |
| 0.434 | | | | | | | 0.07 |
| 0.682 | | | | | | | 0.06 |
| 0.842 | | | | | | | 0.04 |
| 1.032 | | 0.04 | 0.04 | 0.04 | 0.05 | | 0.06 |
| 1.049 | 0.03 | 0.04 | 0.04 | 0.04 | 0.04 | 0.03 | |
| RS-2 | | 0.03 | 0.03 | 0.03 | 0.03 | 0.04 | |
| 1.113 | | | | | | | 0.05 |
| 1.140 | 0.04 | 0.04 | 0.04 | 0.04 | 0.03 | | 0.03 |
| 1.966 | | | | | | | 0.04 |
| **Total** | 0.11 | 0.35 | 0.55 | 0.49 | 0.91 | 0.63 | 1.81 |

### Example 7. Scale-up: 1 kg.

The direct blend and encapsulation process and formulation identified in the previous section was processed at ~ 500 g blend split to make multiple strengths. The blend was manually filled in capsules used for stability testing, thus the next step was to scale-up the blend and evaluate it on an automatic encapsulator. This was executed in a two phase approach. First, a 1 kg blend was generated for the 5 mg dose and for the 50 mg dose per the formulation listed in Table **50** to bracket all strengths.

**Table 50. Formulation table for 5 mg and 50 mg capsules.**

| Ingredient | 5 mg strength | | 50 mg strength | |
|---|---|---|---|---|
| | % w/w | mg/unit | % w/w | mg/unit |
| Compound **A** free base | 5.00 | 5.00 | 25.00 | 50.00 |
| | 8.35* | 8.35* | 41.75* | 83.50* |
| Fumaric acid | 5.00 | 5.00 | 25.00 | 50.00 |
| Lactose anhydrous | 69.50 | 69.50 | 29.50 | 59.00 |
| | 66.15* | 66.15* | 12.75* | 25.50* |
| Avicel DG | 15.00 | 15.00 | 15.00 | 30.00 |
| Crospovidone | 5.00 | 5.00 | 5.00 | 10.00 |
| Magnesium stearate | 0.50 | 0.50 | 0.50 | 1.00 |
| Total | 100.00 | 100.00 | 100.00 | 200.00 |

| **Encapsulation** | | | | |
|---|---|---|---|---|
| Size 2 CS VCaps plus white opaque | | Capsugel; Code: V44.900 | | |

| | | | | |
|---|---|---|---|---|
| *Potency calculation - 1 kg scale-up Potency (%) = (100 - KF - S) × P / 100 × MW Ratio Freebase / Bis-Salt KF = Water content by Karl Fischer = 0.3 (obtained from CofA) S = Total residual solvents = 0.1234 (obtained from CofA) P = % Purity = 99.95% (obtained from CofA) MW = (Molecular weight of free base = 519.63) / (Molecular weight of Bis-Salt = 864.02) Potency (%) = (100-0.3-0.1234) × 99.95 / 100 × 519.63 / 864.02 = 59.8564% Correction factor calculation Correction factor = 100 / Potency = 100 / 59.8564 = 1.67 Potency calculation - 2 kg scale-up Potency (%) = (100 - KF - S) × P / 100 × MW Ratio Freebase / Bis-Salt KF = Water content by Karl Fischer = 0.6 (obtained from CofA) S = Total residual solvents = 0.4090 (obtained from CofA) P = % Purity = 100.0% (obtained from CofA) MW = (Molecular weight of free base = 519.63) / (Molecular weight of Bis-Salt = 864.02) Potency (%) = (100-0.6-0.4090) × 100.0 / 100 × 519.63 / 864.02 = 59.5341% Correction factor calculation Correction factor = 100 / Potency = 100 / 59.5341 = 1.68 | | | | |

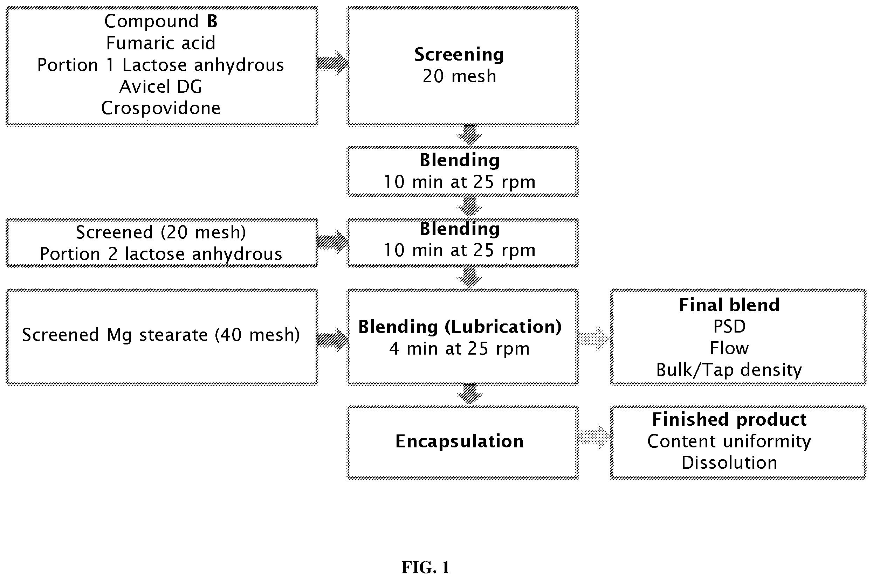

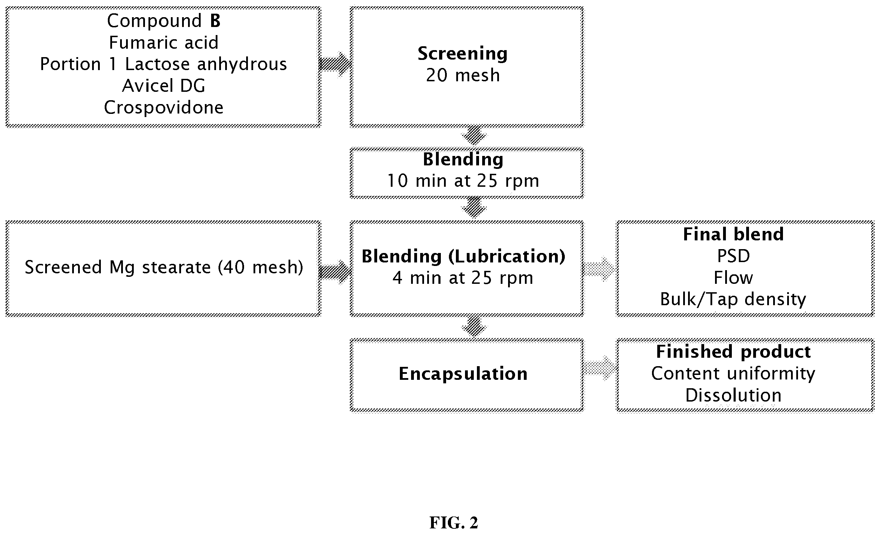

The manufacturing process for both blends is similar and is presented in Figure 1 and Figure 2. Briefly, for the 5 mg blend, Compound **B** was blended with fumaric acid, Avicel DG, Polyplasdone XL, and half the lactose anhydrous DT, for 250 revolutions. The second half of lactose anhydrous DT was added to this blend and mixed for an additional 250 revolutions, followed by blending with magnesium stearate for 100 revolutions. The 50 mg blend was manufactured the same except the lactose was added as one portion with the fumaric acid, Avicel DG, and Polyplasdone XL for 250 revolutions. Magnesium stearate was blended for 100 revolutions. The physical properties of both blends are listed in Table **51.**

**Table 51. Physical properties of blends.**

| **Test** | **5 mg capsules** | **50 mg capsules** |
|---|---|---|
| Bulk density (g / mL) | 0.51 | 0.50 |
| Tapped density (g / mL) | 0.76 | 0.69 |
| Hausner ratio | 1.47 | 1.39 |
| Compressibility index (%) | 32 | 28 |
| Flow using flodex (orifice mm) | 18 | 16 |
| Angle of repose | 42 | 32 |

Both blends were encapsulated on the MG Flexalab automatic encapsulator. The fill weight range for the 5 mg blend was 100 mg ± 5 % equivalent to 95 - 105 mg, and that for the 50 mg blend was 200 mg ± 5 % equivalent to 190 - 210 mg. The average empty size 2 capsule weight was measured at 59.0 mg, thus the 5 mg capsules were targeted to be 159 mg (154 - 164 mg) and the 50 mg capsules were targeted to be 259 mg (149 - 169 mg).

The capsules were processed through a weight sorter and the data generated was processed to eliminate rejects that were related to errors due to the performance of the encapsulator. The acceptance rate for the 5 mg capsules was > 94 %, and for the 50 mg capsules was > 99 %. Thus, the blend seems to be amenable to processing on a high speed encapsulator. Capsules collected at the beginning, middle, and end of encapsulation were tested for content uniformity. The 5 mg capsules had an acceptance value of 4.5 - 6 and the 50 mg capsules had an acceptance value of 1.9 - 3.5; the data are presented in Table **52.** The encapsulation processing parameters are listed in Table **53.**

**Table 52. Encapsulation parameters I.**

| # | 5 mg capsules | | | 50 mg capsules | | |
|---|---|---|---|---|---|---|
| | Beginning 0-20 min % LC | Middle 20-40 min % LC | End 40-60 min % LC | Beginning 0-20 min % LC | Middle 20-40 min % LC | End 40-60 min % LC |
| 1 | 99.7 | 102.8 | 104.1 | 103.4 | 100.6 | 104.2 |
| 2 | 96.4 | 101.4 | 103.5 | 103.7 | 99.4 | 103.9 |
| 3 | 96.8 | 105.4 | 104.4 | 102.7 | 100.6 | 103.4 |
| 4 | 99.9 | 98.3 | 103.1 | 103.4 | 100.1 | 102.3 |
| 5 | 101.4 | 101.7 | 105.3 | 103.0 | 99.8 | 103.9 |
| 6 | 101.4 | 100.8 | 106.2 | 103.6 | 100.2 | 103.4 |
| 7 | 100.9 | 101.6 | 103.9 | 103.3 | 99.9 | 103.5 |
| 8 | 101.6 | 102.4 | 105.5 | 103.2 | 99.5 | 103.3 |
| 9 | 98.2 | 100.8 | 102.9 | 103.2 | 198.0 | 103.7 |
| 10 | 105.0 | 99.5 | 100.2 | 103.8 | 99.6 | 104.3 |
| Mean | 100.1 | 101.5 | 103.9 | 103.3 | 99.8 | 103.6 |
| % RSD | 2.5 | 1.9 | 1.6 | 0.3 | 0.8 | 0.5 |
| Min | 96.4 | 98.3 | 100.2 | 102.7 | 98.0 | 102.3 |
| Max | 105.0 | 105.4 | 106.2 | 103.8 | 100.6 | 104.3 |
| Std. Dev. | 2.5 | 1.9 | 1.7 | 0.3 | 0.8 | 0.6 |
| AV | 6.0 | 4.6 | 4.5 | 2.5 | 1.9 | 3.5 |

**Table 53. Encapsulation parameters II.**

| | 5 mg capsules | 50 mg capsules | 5 mg capsules | 5 mg capsules | 50 mg capsules | 50 mg capsules |
|---|---|---|---|---|---|---|
| Average empty capsule shell weight (n=50) | 59.0 | 58.49 | 58.8 | 59.6 | 58.7 | 59.07 |
| Target fill weight, mg | 100.0 | 200.0 | 100.0 | 100.0 | 200.0 | 200.0 |
| Target filled capsule weight, mg | 159.0 | 258.5 | 158.8 | 159.6 | 258.7 | 259.07 |
| ±5% range for fill weight, mg | 95.0 - 105.0 | 190.0 - 210.0 | 95.0 - 105 | 95.0 - 105 | 190.0 - 210.0 | 190.0 - 210.0 |
| ±5% filled capsule weight range, mg | 154.0 - 164.0 | 248.5 - 268.5 | 153.8 - 163.8 | 154.6 - 164.6 | 248.7 - 268.7 | 249.07 - 269.07 |
| Size 2 Vcaps plus closed length specification (per Capsugel), mm | 17.70 - 18.30 | 17.70 - 18.30 | 17.70 - 18.30 | 17.70 - 18.30 | 17.70 - 18.30 | 17.70 - 18.30 |

| Encapsulator parameters | | | | | | |
|---|---|---|---|---|---|---|
| Set Up parameters | 5 mg capsules | 50 mg capsules | 5 mg capsules | 5 mg capsules | 50 mg capsules | 50 mg capsules |
| Dosator | Size 3 | Size 2 | Size 3 | Size 3 | Size 2 | Size 2 |
| Dosator height, mm | 7 | 12 | 7 | 7 | 12 | 12 |
| Compression head, mm | 4 | 10 | 4 | 4 | 10 | 10 |
| Compaction / compression, mm | 3 | 2 | 3 | 3 | 2 | 2 |
| Encapsulator speed, capsules / h | ~1500 | ~1000 | 2250 - 2500 | 2250 | 2000 | 2000 |
| Powder bed depth / height, mm | 16 | 39 | 16 | 16 | 39 | 39 |

### Example 8. Scale-up: 2 kg.

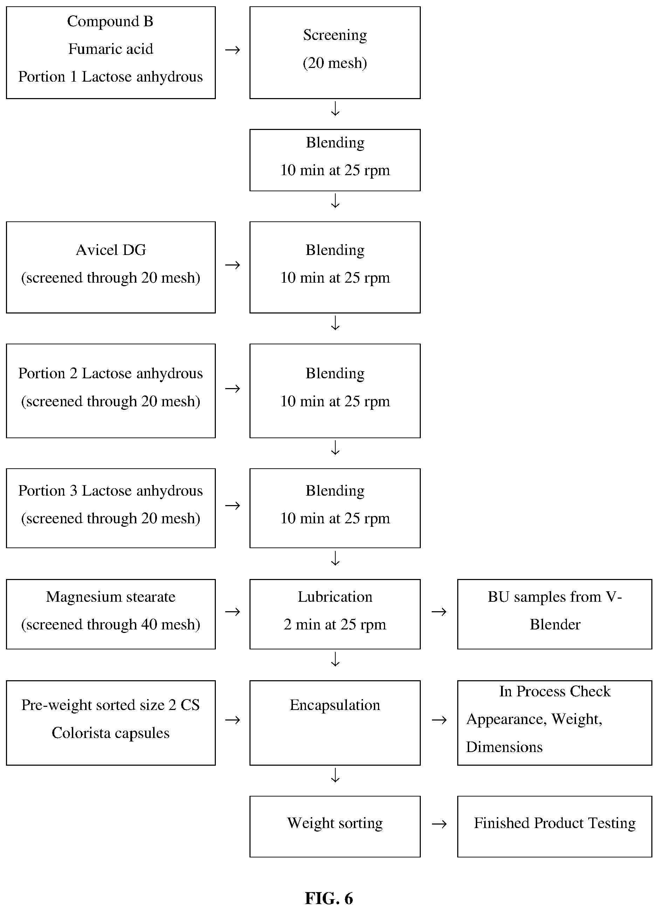

The first scale-up to 1 kg was deemed successful based off the AV values observed for the 5 mg dose and the 50 mg dose. The second phase of scale-up was executed at a 2 kg scale for the 5 mg dose and 50 mg dose. Each blend was encapsulated in size 2 white opaque capsules and in COLORISTA^{®} all-color capsules. The formulation details are listed in Table **50,** and the manufacturing process is presented in FIG. 1, FIG. 2, and FIG. 6. The encapsulation processing parameters are listed in Table **53.**

Each formulation was placed on stability at 25°C / 60 % RH and 50°C / 75% RH for up to 16 weeks. The rationale was to evaluate stability of the encapsulated blend post processing through a complete manufacturing process and comparing the trend in RS between the white capsules and the COLORISTA^{®} capsules.

Both white and COLORISTA^{®} capsules formulated with 5 mg or 50 mg of Compound **B** were packaged in bottles as well as blisters and placed on stability at 25°C / 60% RH and 50°C / 75 % RH for 16 weeks in the following configurations:
5 mg capsules (white opaque) in bottle; 20 capsules per bottle
5 mg capsules (white opaque) in blister; 6 capsules per blister
5 mg capsules (COLORISTA^{®}) in bottle; 20 capsules per bottle
5 mg capsules (COLORISTA^{®}) in blister; 6 capsules per blister
50 mg capsules (white opaque) in bottle; 20 capsules per bottle
50 mg capsules (white opaque) in blister; 6 capsules per blister
50 mg capsules (COLORISTA^{®}) in bottle; 20 capsules per bottle
50 mg capsules (COLORISTA^{®}) in blister; 6 capsules per blister

The following packaging materials were used:
Bottles: 30 cc HDPE bottles, wide mouth pharmaceutical round white bottle
Caps: 28 mm SecuRx RbTx white FSM1 w / .035 pupl Prt 'SFYP' Wht
Blister material: ALU-ALU blister

Stratified content uniformity test was performed on capsules collected throughout the encapsulation run. The individual capsule assay values for Compound **A,** 5 mg capsules, was in the range of 96.6 - 108.9 % (Table **55).** This data was verified with a composite sample collected at the end of the run. The blend uniformity results are listed in Table **56** and are in the range of 100.5 - 103.8 %. The higher variation in content uniformity as compared to blend uniformity was attributed to the variation observed in empty capsule shell weights. The average size 2 white opaque VCaps plus empty capsule weight was reported at ~59 mg with a distribution from 50 mg - 65 mg (Table **57).** The fill weight for the 5 mg capsules is listed at 100 mg with a range of 95 - 105 mg. Thus, the empty capsule weight varies up to 15 mg and the filled capsule weight needs to be maintained within 10 mg. A proposed solution for future batches is to use a smaller capsule size and potentially weight sort the empty capsules to a narrow weight range to avoid such high variances in content uniformity testing. The content uniformity results for 50 capsules are listed in Table **54.** These present significantly lower variation within individual capsule assay values due to the higher fill weight and permissible variation within the blend.

**Table 54. Uniformity for 5 mg and 50 mg batches.**

| # | 5 mg capsules | 50 mg capsules |
|---|---|---|
| | % | % |
| 1 | 101.1 | 100.7 |
| 2 | 100.5 | 100.8 |
| 3 | 100.7 | 101.2 |
| 4 | 103.8 | 98.3 |
| 5 | 101.1 | 101.2 |
| Mean | 101.4 | 100.4 |
| %RSD | 1.3 | 1.2 |
| Min | 100.5 | 98.3 |
| Max | 103.8 | 101.2 |

**Table 55. Content uniformity for 5 mg capsules: scale up batch 2 kg.**

| **#** | **% LC** | | | | | | | |
|---|---|---|---|---|---|---|---|---|
| | O - B* | O - M* | O - E* | **Composite** | C - B* | C - M* | C - E* | **Composite** |
| 1 | 99.1 | 105.3 | 97.2 | 104.4 | 96.6 | 108.9 | 106.6 | 102.9 |
| 2 | 99.7 | 104.6 | 105.3 | 99.3 | 98.5 | 103.4 | 105.9 | 106.3 |
| 3 | 102.5 | 99.2 | 99.7 | 102.4 | 100.1 | 104.2 | 104.2 | 104.9 |
| 4 | 102.7 | 107.3 | 99.3 | 106.6 | 100.0 | 102.1 | 103.5 | 105.6 |
| 5 | 103.7 | 100.5 | 101.6 | 99.3 | 101.5 | 108.1 | 105.4 | 100.3 |
| 6 | 104.4 | 102.4 | 100.0 | 100.9 | 99.7 | 101.9 | 105.0 | 106.0 |
| | | | | 100.4 | | | | 102.4 |
| | | | | 97.7 | | | | 101.0 |
| | | | | 103.9 | | | | 95.3 |
| | | | | 99.7 | | | | 101.1 |
| **Avg** | 102.0 | 103.2 | 100.5 | 101.5 | 99.4 | 104.8 | 105.1 | 102.6 |
| **% RSD** | 2.1 | 3.0 | 2.7 | 2.7 | 1.7 | 2.9 | 1.1 | 3.3 |
| **Min** | 99.1 | 99.2 | 97.2 | 97.7 | 96.6 | 101.9 | 103.5 | 95.3 |
| **Max** | 104.4 | 107.3 | 105.3 | 106.6 | 101.5 | 108.9 | 106.6 | 106.3 |
| **Std. Dev.** | 2.2 | 3.1 | 2.7 | 2.8 | 1.7 | 3.0 | 1.1 | 3.4 |
| **AV value** | | | | 6.7 | | | | 9.2 |

| | | | | | | | | |
|---|---|---|---|---|---|---|---|---|
| O - B: white opaque capsules; beginning of encapsulation run O - M: white opaque capsules; middle of encapsulation run O - E: white opaque capsules; end of encapsulation run C - B: COLORISTA^{®}; beginning of encapsulation run C - M: COLORISTA^{®}; middleof encapsulation run C - E: COLORISTA^{®}; end of encapsulation run | | | | | | | | |

**Table 56. Content uniformity for 50 mg capsules.**

| **#** | **%LC** |
|---|---|
| 1 | 99.0 |
| 2 | 100.2 |
| 3 | 101.4 |
| 4 | 99.1 |
| 5 | 99.7 |
| 6 | 101.9 |
| 7 | 101.0 |
| 8 | 100.6 |
| 9 | 98.5 |
| 10 | 99.8 |
| 11 | 99.1 |
| 12 | 99.0 |
| 13 | 100.6 |
| 14 | 100.0 |
| 15 | 99.1 |
| 16 | 100.1 |
| 17 | 99.9 |
| 18 | 100.9 |
| 19 | 101.5 |
| 20 | 100.1 |
| 21 | 100.1 |
| 22 | 101.5 |
| 23 | 98.9 |
| 24 | 100.9 |
| 25 | 100.3 |
| 26 | 99.3 |
| 27 | 101.3 |
| 28 | 100.3 |
| 29 | 101.5 |
| 30 | 99.2 |
| **Avg** | 100.2 |
| **%RSD** | 1.0 |
| **Min** | 98.5 |
| **Max** | 101.9 |
| **Std. Dev.** | 1.0 |
| **AV** | 2.0 |

**Table 57. Weight sorting 2 white opaque Vcaps plus - empty capsule shells.**

| **# of capsules** | **Weight Range** | **%** |
|---|---|---|
| 191 | 50-57 mg | 18.41 |
| 352 | 57-61 mg | 33.84 |
| 494 | 61-65 mg | 47.50 |
| 3 | 65 - 100 mg | 0.28 |

The assay value for 5 mg capsules presents significant variation at individual time points tested. This is expected due to the variance observed in the individual capsule assay values during content uniformity testing. The assay value for 50 mg capsules was within expected variance during the 16 week stability study. **RS-1** was maintained in the 0.06 - 0.07 % range through the 16 weeks for all formulations tested. The results from all samples are listed in Table **58** - Table **73.**

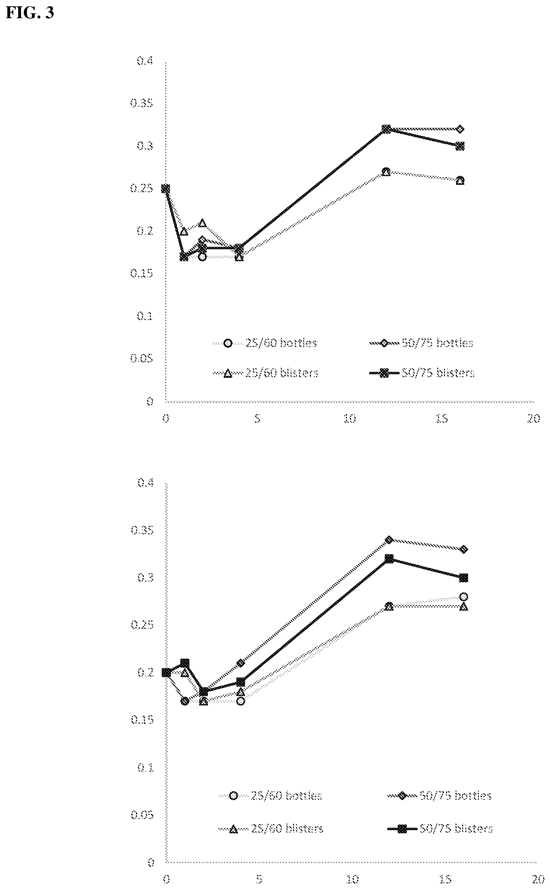

The total RS appear to be out of trend during this study for both strengths. The data suggests that the total RS initially dropped from ~0.20 % - 0.25 % to ∼0.16 - 0.17 % at t = 1 week, and remained at that level up to ~ 4 weeks in all samples and temperature conditions tested. It is noted that the individual RS for this study were integrated at level >LOD but <LOQ in order to trend if these indeed grow over time or disappear. The peak at ~RRT 1.922 was below LOQ and disappeared after t = 0, which explains the trend of drop in RS. However, at t = 12 weeks there are new RS observed in each formulation and packaging configuration that contribute to the total RS being higher. This higher level of RS is observed at both 25°C / 60 % RH and 50°C / 75 % RH conditions at relatively similar levels and are retained through the 16 week time point. The packaging configuration did not impact the total RS for the duration of this study. It is noted that in all cases the known **RS-1** is relatively unaffected by exposure to time and temperature. The trend of total RS over time are presented in Figure 3.

**Table 58. Stability profile for 5 mg white capsules in bottles; 25 °C, 60% RH.**

| **t, week** | 0 | 1 | 2 | 4 | 12 | 16 |
|---|---|---|---|---|---|---|
| **% LC** | 100.4 | 104.2 | 102.2 | 99.8 | 103.3 | 0 |
| **Name / RRT** | | | | | | |
| RS-1 | 0.07 | 0.06 | 0.07 | 0.07 | 0.07 | 0.07 |
| RS-2 | | | | | 0.04 | 0.03 |
| 0.283 | | | | | | |
| 1.048 | | | | | 0.05 | 0.05 |
| 1.072 | 0.06 | 0.06 | 0.06 | 0.06 | 0.08 | 0.08 |
| 1.117 | 0.04 | 0.04 | 0.04 | 0.04 | 0.04 | 0.04 |
| 1.579 | 0.05 | | | | | |
| 1.922 | 0.03 | | | | | |
| **Total** | 0.25 | 0.17 | 0.17 | 0.17 | 0.27 | 0.26 |
| **% Moisture** | | | | | 1.7 | 1.7 |

**Table 59. Stability profile for 5 mg white capsules in bottles; 50 °C, 75% RH.**

| **t, week** | 0 | 1 | 2 | 4 | 12 | 16 |
|---|---|---|---|---|---|---|
| **% LC** | 100.4 | 100.7 | 102 | 103.3 | 101.5 | 0 |
| **Name / RRT** | | | | | | |
| RS-1 | 0.07 | 0.07 | 0.07 | 0.08 | 0.07 | 0.08 |
| RS-2 | | | | | 0.04 | 0.03 |
| 0.283 | | | | | 0.05 | 0.05 |
| 1.048 | | | | | 0.05 | 0.04 |
| 1.072 | 0.06 | 0.06 | 0.07 | 0.06 | 0.08 | 0.08 |
| 1.117 | 0.04 | 0.04 | 0.05 | 0.04 | 0.04 | 0.04 |
| 1.579 | 0.05 | | | | | |
| 1.922 | 0.03 | | | | | |
| **Total** | 0.25 | 0.17 | 0.19 | 0.18 | 0.32 | 0.32 |
| **% Moisture** | | | | | 1.7 | 1.8 |

**Table 60. Stability data for 5 mg COLORISTA^{®} capsules in bottles; 25 °C, 60% RH.**

| **t, week** | 0 | 1 | 2 | 4 | 12 | 16 |
|---|---|---|---|---|---|---|
| **% LC** | 99.6 | 104.3 | 102.8 | 102.9 | 104.4 | 0 |
| **Name / RRT** | | | | | | |
| RS-1 | 0.06 | 0.06 | 0.07 | 0.07 | 0.07 | 0.07 |
| RS-2 | | | | | 0.03 | 0.03 |
| 0.289 | | | | | | |
| 1.048 | | | | | 0.05 | 0.05 |
| 1.072 | 0.06 | 0.06 | 0.06 | 0.06 | 0.07 | 0.08 |
| 1.117 | 0.04 | 0.04 | 0.04 | 0.04 | 0.05 | 0.04 |
| 1.922 | 0.03 | | | | | |
| **Total** | 0.20 | 0.17 | 0.17 | 0.17 | 0.27 | 0.28 |
| **% Moisture** | | | | | 1.7 | 1.7 |

**Table 61. Stability data for 5 mg COLORISTA^{®} capsules in bottles; 50 °C, 75% RH.**

| **t, week** | 0 | 1 | 2 | 4 | 12 | 16 |
|---|---|---|---|---|---|---|
| **% LC** | 99.6 | 104.8 | 103.2 | 101.4 | 104 | 0 |
| **Name / RRT** | | | | | | |
| RS-1 | 0.06 | 0.07 | 0.07 | 0.07 | 0.07 | 0.08 |
| RS-2 | | | | | 0.04 | 0.04 |
| 0.289 | | | | 0.04 | 0.06 | 0.05 |
| 1.048 | | | | | 0.04 | 0.04 |
| 1.072 | 0.06 | 0.06 | 0.06 | 0.06 | 0.08 | 0.08 |
| 1.117 | 0.04 | 0.04 | 0.04 | 0.04 | 0.04 | 0.04 |
| 1.922 | 0.03 | | | | | |
| **Total** | 0.20 | 0.17 | 0.18 | 0.21 | 0.34 | 0.33 |
| **% Moisture** | | | | | 1.7 | 1.8 |

**Table 62. Stability data for 50 mg white capsules in in bottles; 25 °C, 60% RH.**

| | | | | | | |
|---|---|---|---|---|---|---|
| **t, week** | 0 | 1 | 2 | 4 | 12 | 16 |
| **% LC** | 99.9 | 103 | 101.2 | 100.8 | 100.6 | 0 |

| **Name / RRT** | | | | | | |
|---|---|---|---|---|---|---|
| RS-1 | 0.06 | 0.07 | 0.06 | 0.07 | 0.07 | 0.06 |
| RS-2 | | | | | 0.03 | 0.03 |
| 1.048 | | | | | 0.06 | 0.05 |
| 1.072 | 0.06 | 0.06 | 0.06 | 0.06 | 0.08 | 0.07 |
| 1.117 | 0.04 | 0.04 | 0.04 | 0.04 | 0.04 | 0.04 |
| 1.927 | 0.03 | 0.04 | 0.03 | | | |
| **Total** | 0.20 | 0.21 | 0.2 | 0.17 | 0.27 | 0.26 |
| **% Moisture** | | | | | 2.2 | 2.1 |

**Table 63. Stability data for 50 mg white capsules in in bottles; 50 °C, 75% RH.**

| | | | | | | |
|---|---|---|---|---|---|---|
| **t, week** | 0 | 1 | 2 | 4 | 12 | 16 |
| **% LC** | 99.9 | 103 | 100.5 | 100.3 | 101.4 | 0 |

| **Name / RRT** | | | | | | |
|---|---|---|---|---|---|---|
| RS-1 | 0.06 | 0.07 | 0.07 | 0.07 | 0.07 | 0.07 |
| RS-2 | | | | | 0.04 | 0.03 |
| 1.048 | | | | | 0.05 | 0.04 |
| 1.072 | 0.06 | 0.06 | 0.07 | 0.06 | 0.07 | 0.08 |
| 1.117 | 0.04 | 0.04 | 0.04 | 0.04 | 0.04 | 0.04 |
| 1.927 | 0.03 | | | | | |
| **Total** | 0.20 | 0.18 | 0.18 | 0.18 | 0.27 | 0.26 |
| **% Moisture** | | | | | 2.4 | 1.8 |

**Table 64. Stability data for 50 mg COLORISTA^{®} capsules in bottles; 25 °C, 60% RH.**

| | | | | | | |
|---|---|---|---|---|---|---|
| **t, week** | 0 | 1 | 2 | 4 | 12 | 16 |
| **% LC** | 100.1 | 101.8 | 100.7 | 101.6 | 102 | 0 |

| **Name / RRT** | | | | | | |
|---|---|---|---|---|---|---|
| RS-1 | 0.06 | 0.06 | 0.06 | 0.06 | 0.07 | 0.06 |
| RS-2 | | | | | 0.04 | 0.03 |
| 1.048 | | | | | 0.06 | 0.05 |
| 1.072 | 0.06 | 0.06 | 0.06 | 0.07 | 0.08 | 0.07 |
| 1.118 | 0.04 | 0.04 | 0.04 | 0.04 | 0.04 | 0.04 |
| 1.923 | 0.04 | | | | | |
| **Total** | 0.20 | 0.16 | 0.17 | 0.17 | 0.28 | 0.25 |
| **% Moisture** | | | | | 2.3 | 2.2 |

**Table 65. Stability data for 50 mg COLORISTA^{®} capsules in bottles; 50 °C, 75% RH.**

| | | | | | | |
|---|---|---|---|---|---|---|
| **t, week** | 0 | 1 | 2 | 4 | 12 | 16 |
| **% LC** | 100.1 | 101.4 | 101.9 | 102.7 | 100.1 | 0 |

| **Name / RRT** | | | | | | |
|---|---|---|---|---|---|---|
| RS-1 | 0.06 | 0.06 | 0.07 | 0.07 | 0.07 | 0.07 |
| RS-2 | | | | | 0.03 | 0.03 |
| 1.048 | | | | | 0.05 | 0.04 |
| 1.072 | 0.06 | 0.06 | 0.06 | 0.07 | 0.07 | 0.07 |
| 1.118 | 0.04 | 0.04 | 0.04 | 0.04 | 0.04 | 0.04 |
| 1.923 | 0.04 | | | | | |
| **Total** | 0.20 | 0.17 | 0.17 | 0.18 | 0.26 | 0.25 |
| **% Moisture** | | | | | 2.3 | 1.8 |

**Table 66. Stability data for 5 mg white capsules in blisters; 25 °C, 60 % RH.**

| | | | | | | |
|---|---|---|---|---|---|---|
| **t, week** | 0 | 1 | 2 | 4 | 12 | 16 |
| **% LC** | 100.4 | 99.6 | 100.6 | 99.8 | 101.1 | 0 |

| **Name / RRT** | | | | | | |
|---|---|---|---|---|---|---|
| RS-1 | 0.07 | 0.07 | 0.06 | 0.06 | 0.07 | 0.07 |
| RS-2 | | | | | 0.04 | 0.03 |
| 1.048 | | | | | 0.05 | 0.05 |
| 1.072 | 0.06 | 0.06 | 0.06 | 0.06 | 0.07 | 0.07 |
| 1.117 | 0.04 | 0.04 | 0.04 | 0.05 | 0.04 | 0.04 |
| 1.579 | 0.05 | 0.04 | 0.04 | | | |
| 1.922 | 0.03 | | | | | |
| **Total** | 0.25 | 0.2 | 0.21 | 0.17 | 0.27 | 0.26 |
| **% Moisture** | | | | | 1.5 | 1.5 |

**Table 67. Stability data for 5 mg white capsules in blisters; 50 °C, 75 % RH.**

| | | | | | | |
|---|---|---|---|---|---|---|
| **t, week** | 0 | 1 | 2 | 4 | 12 | 16 |
| **% LC** | 100.4 | 103.3 | 102.4 | 101.3 | 103.2 | 0 |

| **Name / RRT** | | | | | | |
|---|---|---|---|---|---|---|
| RS-1 | 0.07 | 0.07 | 0.07 | 0.07 | 0.07 | 0.07 |
| RS-2 | | | | | 0.04 | 0.03 |
| 0.283 | | | | | 0.04 | 0.05 |
| 1.048 | | | | | 0.05 | 0.05 |
| 1.072 | 0.06 | 0.06 | 0.06 | 0.06 | 0.08 | 0.07 |
| 1.117 | 0.04 | 0.04 | 0.04 | 0.04 | 0.04 | 0.04 |
| 1.579 | 0.05 | | | | | |
| 1.922 | 0.03 | | | | | |
| **Total** | 0.25 | 0.17 | 0.18 | 0.18 | 0.32 | 0.3 |
| **% Moisture** | | | | | 1.4 | 1.3 |

**Table 68. Stability data for 5 mg COLORISTA^{®} capsules in blisters; 25 °C, 60 % RH.**

| | | | | | | |
|---|---|---|---|---|---|---|
| **t, week** | 0 | 1 | 2 | 4 | 12 | 16 |
| **% LC** | 99.6 | 101.4 | 103.8 | 103.9 | 104.1 | 0 |

| **Name / RRT** | | | | | | |
|---|---|---|---|---|---|---|
| RS-1 | 0.06 | 0.07 | 0.07 | 0.07 | 0.07 | 0.07 |
| RS-2 | | | | | 0.04 | 0.03 |
| 0.283 | | | | | | |
| 1.048 | | | | | 0.05 | 0.05 |
| 1.072 | 0.06 | 0.06 | 0.06 | 0.07 | 0.07 | 0.08 |
| 1.117 | 0.04 | 0.04 | 0.04 | 0.05 | 0.04 | 0.04 |
| 1.922 | 0.03 | 0.03 | | | | |
| **Total** | 0.20 | 0.2 | 0.17 | 0.18 | 0.27 | 0.27 |
| **% Moisture** | | | | | 1.5 | 1.5 |

**Table 69. Stability data for 5 mg COLORISTA^{®} capsules in blisters; 50 °C, 75 % RH.**

| | | | | | | |
|---|---|---|---|---|---|---|
| **t, week** | 0 | 1 | 2 | 4 | 12 | 16 |
| **% LC** | 99.6 | 104.1 | 103.4 | 104.8 | 102.7 | 0 |

| **Name / RRT** | | | | | | |
|---|---|---|---|---|---|---|
| RS-1 | 0.06 | 0.1 | 0.07 | 0.08 | 0.07 | 0.07 |
| RS-2 | | | | | 0.04 | 0.03 |
| 0.283 | | | | | 0.05 | 0.05 |
| 1.048 | | | | | 0.05 | 0.04 |
| 1.072 | 0.06 | 0.06 | 0.07 | 0.07 | 0.07 | 0.07 |
| 1.117 | 0.04 | 0.04 | 0.04 | 0.05 | 0.04 | 0.04 |
| 1.922 | 0.03 | | | | | |
| **Total** | 0.20 | 0.21 | 0.18 | 0.19 | 0.32 | 0.3 |
| **% Moisture** | | | | | 1.4 | 1.3 |

**Table 70. Stability data for 50 mg white capsules in blisters; 25 °C, 60 % RH.**

| **t, week** | 0 | 1 | 2 | 4 | 12 | 16 |
|---|---|---|---|---|---|---|
| **% LC** | 99.9 | 101.3 | 100.7 | 101.9 | 102.4 | 0 |

| **Name / RRT** | | | | | | |
|---|---|---|---|---|---|---|
| RS-1 | 0.06 | 0.06 | 0.06 | 0.07 | 0.07 | 0.06 |
| RS-2 | | | | | 0.04 | 0.03 |
| 1.048 | | | | | 0.05 | 0.06 |
| 0.143 | | | | | | |
| 1.072 | 0.06 | 0.06 | 0.06 | 0.06 | 0.08 | 0.08 |
| 1.117 | 0.04 | 0.04 | 0.04 | 0.04 | 0.04 | 0.04 |
| 1.927 | 0.03 | 0.04 | 0.03 | | | |
| **Total** | 0.20 | 0.2 | 0.2 | 0.17 | 0.28 | 0.27 |
| **% Moisture** | | | | | 2.0 | 2.0 |

**Table 71. Stability data for 50 mg white capsules in blisters; 50 °C, 75 % RH.**

| **t, week** | 0 | 1 | 2 | 4 | 12 | 16 |
|---|---|---|---|---|---|---|
| **% LC** | 99.9 | 102.8 | 100.6 | 100.7 | 101.5 | 0 |

| **Name / RRT** | | | | | | |
|---|---|---|---|---|---|---|
| RS-1 | 0.06 | 0.07 | 0.07 | 0.07 | 0.07 | 0.07 |
| RS-2 | | | | | 0.03 | 0.03 |
| 1.048 | | | | | 0.05 | 0.04 |
| 0.143 | | 0.08 | | | | |
| 1.072 | 0.06 | 0.06 | 0.06 | 0.06 | 0.06 | 0.08 |
| 1.117 | 0.04 | 0.04 | 0.04 | 0.04 | 0.04 | 0.04 |
| 1.927 | 0.03 | | | | | |
| **Total** | 0.20 | 0.25 | 0.18 | 0.18 | 0.26 | 0.26 |
| **% Moisture** | | | | | 1.9 | 1.5 |

**Table 72. Stability data for 50 mg COLORISTA^{®} capsules in blisters; 25 °C, 60 % RH.**

| **t, week** | 0 | 1 | 2 | 4 | 12 | 16 |
|---|---|---|---|---|---|---|
| **% LC** | 100.1 | 100.4 | 101.2 | 101.9 | 101.1 | 0 |

| **Name / RRT** | | | | | | |
|---|---|---|---|---|---|---|
| RS-1 | 0.06 | 0.07 | 0.06 | 0.07 | 0.07 | 0.07 |
| RS-2 | | | | | 0.04 | 0.03 |
| 1.049 | | | | | 0.06 | 0.05 |
| 1.072 | 0.06 | 0.06 | 0.06 | 0.07 | 0.07 | 0.07 |
| 1.118 | 0.04 | 0.04 | 0.04 | 0.04 | 0.04 | 0.04 |
| 1.549 | | | 0.04 | | | |
| 1.923 | 0.04 | 0.03 | | | | |
| **Total** | 0.20 | 0.2 | 0.21 | 0.18 | 0.28 | 0.26 |
| **% Moisture** | | | | | 2.1 | 2.0 |

**Table 73. Stability data for 50 mg COLORISTA^{®} capsules in blisters; 50 °C, 75 % RH.**

| **t, week** | 0 | 1 | 2 | 4 | 12 | 16 |
|---|---|---|---|---|---|---|
| **% LC** | 100.1 | 102 | 101 | 101.1 | 102.4 | 0 |

| **Name / RRT** | | | | | | |
|---|---|---|---|---|---|---|
| RS-1 | 0.06 | 0.07 | 0.07 | 0.07 | 0.06 | 0.06 |
| RS-2 | | | | | | 0.03 |
| 1.049 | | | | | 0.05 | 0.05 |
| 1.072 | 0.06 | 0.06 | 0.06 | 0.06 | 0.07 | 0.08 |
| 1.118 | 0.04 | 0.04 | 0.04 | 0.04 | 0.04 | 0.04 |
| 1.549 | | | | | | |
| 1.923 | 0.04 | | | | | |
| **Total** | 0.20 | 0.17 | 0.17 | 0.18 | 0.26 | 0.26 |
| **% Moisture** | | | | | 1.8 | 1.5 |

### Example 9. Crospovidone-free compositions

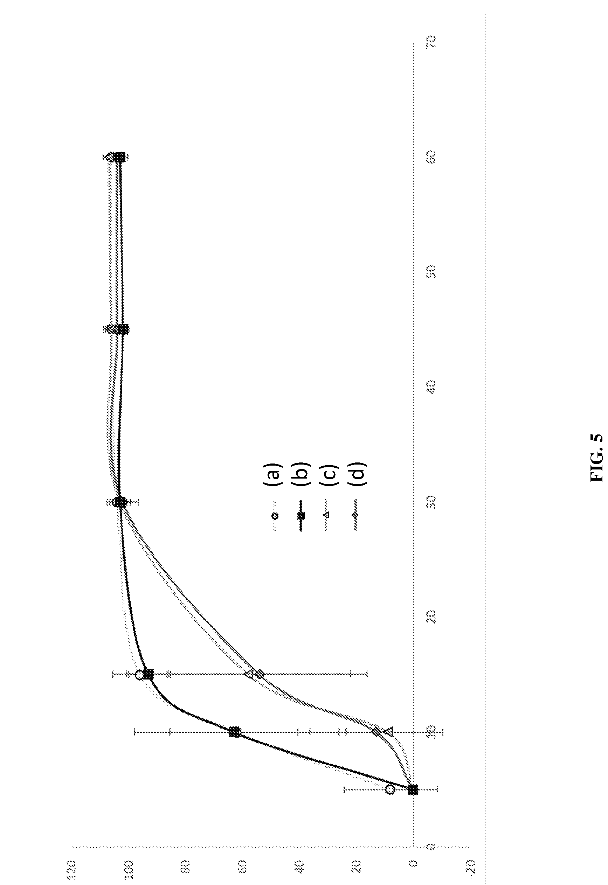

Compound B capsules (5 mg) were manufactured at 300 g batch size without using crospovidone in the formulation. The final blend was manually encapsulated in size 2 CS Vcaps plus COLORISTA^{®} capsules, and n=6 capsules were evaluated for dissolution testing. Table **74** presents formulation information. Capsules were prepared with either CAPSUGEL ^{®} Size 2 CS VCaps plus white opaque capsules or CAPSUGEL ^{®} Size 2 CS VCaps plus COLORISTA^{®} capsules. Table **75** presents dissolution information for 5 mg opaque capsules and 5 mg Table **76** presents dissolution information for 5 mg opaque COLORISTA^{®} capsules, with and without crospovidone. Further dissolution information for white and COLORISTA^{®} capsules is presented in Tables **77** and **78,** respectively, and graphically in FIG. 5.

**Table 74. Formulation information for 5 mg capsules without crospovidone.**

| Ingredient | % w/w | 5 mg strength mg / unit |
|---|---|---|
| Compound **B** | 5.00 (8.5)1 | 5.00 (8.5)1 |
| Fumaric acid Emprove^{®} Essential Cat #8.17073.1000NF, JPE | 5.00 | 5.00 |
| Lactose Anhydrous DT NF, Ph. Eur., JP, BP | 74.50 (71.00)¹ | 74.50 (71.00)¹ |
| Avicel^{®}DG Avicel^{®} Dry Granulation Excipient (Dibasic Calcium Phosphate USP, Ph. Eur., JP, FCC; Microcrystalline Cellulose NF, Ph. Eur., JP) | 15.00 | 15.00 |
| Magnesium stearate, Hyqual^{®}, Vegetable Source, NF-GenAR^{®}, BP, EP, FCC, JP Material #2257-06 | 0.50 | 0.50 |
| Total | 100.00 | 100.00 |

| | | |
|---|---|---|
| ¹Represents potency and salt correction applied to batch quantities *Potency calculation for IMG-7289 API Lot #IMG-7289-0-A-4RP Potency (%) = (100 - KF - S) × P/100 × MW Ratio Freebase/Bis-Salt KF = Water content by Karl Fischer = 1.82 (Ref: C3344-67) S = Total residual solvents = 0.4090 (obtained from CofA for Lot # IMG-7289-0-A-4RP) P = % Purity = 100.0% (obtained from CofA for Lot # IMG-7289-0-A-4RP) MW = (Molecular weight of free base = 519.63)/(Molecular weight of Bis-Salt = 864.02) Potency (%) = (100-1.82-0.4090) × 100.0/100 × 519.63/864.02 = 58.8004% Correction factor calculation Correction factor = 100/Potency = 100/58.8004 = 1.70 | | |

**Table 75. Dissolution of 5 mg white opaque capsules. I.**

| **Time, min** | 15 | 30 | 45 | 60 |
|---|---|---|---|---|
| **#** | | | | |
| 1 | 92.45 | 102.89 | 103.55 | 102.99 |
| 2 | 101.95 | 104.12 | 102.07 | 102.67 |
| 3 | 104.70 | 105.75 | 104.17 | 103.16 |
| 4 | 83.39 | 101.84 | 101.32 | 99.24 |
| 5 | 106.08 | 108.39 | 106.57 | 105.13 |
| 6 | 98.83 | 102.38 | 100.20 | 98.68 |
| **Mean** | 97.9 | 104.23 | 102.98 | 101.98 |
| **% RSD** | 8.79 | 2.37 | 2.21 | 2.45 |

**Table 76. Dissolution of 5 mg COLORISTA^{®} capsules. I.**

| **Crospovidone?** | Y | | | | N | | | |
|---|---|---|---|---|---|---|---|---|
| **Time, min** | 15 | 30 | 45 | 60 | 15 | 30 | 45 | 60 |
| # | | | | | | | | |
| 1 | 1.66 | 92.56 | 100.76 | 101.09 | 64 | 91 | 97 | 98 |
| 2 | 92.2 | 100.71 | 101.75 | 101.87 | 92 | 100 | 100 | 100 |
| 3 | 76.6 | 105.26 | 107.04 | 106.72 | 91 | 97 | 97 | 97 |
| 4 | 92.02 | 101.21 | 102.29 | 102 | 5 | 98 | 106 | 108 |
| 5 | 56.27 | 100.98 | 104.06 | 103.89 | 50 | 102 | 106 | 107 |
| 6 | 85.15 | 97.33 | 100.35 | 100.77 | 98 | 103 | 103 | 103 |
| **Mean** | 67.32 | 99.68 | 102.71 | 102.72 | 67 | 98 | 101 | 102 |
| **% RSD** | 51.74 | 4.31 | 2.42 | 2.18 | 53.3 | 4.3 | 4.3 | 4.5 |

**Table 77. Dissolution of 5 mg white capsules. II.**

| **Crospovidone?** | Y | | | | | | N | | | | | |
|---|---|---|---|---|---|---|---|---|---|---|---|---|
| **Time** | 5 | 10 | 15 | 30 | 45 | 60 | 5 | 10 | 15 | 30 | 45 | 60 |
| | **% drug release** | | | | | | | | | | | |
| # | | | | | | | | | | | | |
| 1 | 41 | 98 | 106 | 106 | 107 | 106 | 0 | 24 | 82 | 105 | 104 | 105 |
| 2 | 0 | 0 | 79 | 100 | 101 | 101 | 0 | 59 | 87 | 99 | 100 | 102 |
| 3 | 6 | 60 | 101 | 106 | 106 | 106 | 0 | 55 | 97 | 103 | 101 | 101 |
| 4 | 0 | 47 | 92 | 102 | 105 | 107 | 0 | 82 | 102 | 104 | 104 | 104 |
| 5 | 0 | 80 | 98 | 106 | 109 | 110 | 0 | 83 | 100 | 104 | 103 | 104 |
| 6 | 0 | 89 | 100 | 105 | 107 | 108 | 0 | 76 | 93 | 101 | 102 | 103 |
| **Mean** | 8 | 62 | 96 | 104 | 106 | 106 | 0 | 63 | 93 | 103 | 102 | 103 |
| **Max** | 41 | 98 | 106 | 106 | 109 | 110 | 0 | 83 | 102 | 105 | 104 | 105 |
| **Min** | 0 | 0 | 79 | 100 | 101 | 101 | 0 | 24 | 82 | 99 | 100 | 101 |
| **% RSD** | 208.6 | 57.4 | 9.8 | 2.4 | 2.4 | 2.5 | 0 | 35.8 | 8.2 | 2.2 | 1.5 | 1.5 |

**Table 78. Dissolution of 5 mg COLORISTA^{®} capsules. II.**

| **Crospovidone?** | Y | | | | | | N | | | | | |
|---|---|---|---|---|---|---|---|---|---|---|---|---|
| **Time** | 5 | 10 | 15 | 30 | 45 | 60 | 5 | 10 | 15 | 30 | 45 | 60 |
| | **% drug release** | | | | | | | | | | | |
| **#** | | | | | | | | | | | | |
| 1 | 0 | 20 | 96 | 105 | 106 | 106 | 0 | 0 | 53 | 99 | 100 | 100 |
| 2 | 0 | 2 | 84 | 104 | 106 | 106 | 0 | 0 | 1 | 94 | 100 | 100 |
| 3 | 0 | 0 | 15 | 99 | 107 | 108 | 0 | 60 | 90 | 108 | 108 | 108 |
| 4 | 0 | 35 | 97 | 106 | 107 | 107 | 0 | 10 | 40 | 101 | 101 | 105 |
| 5 | 0 | 0 | 1 | 97 | 106 | 106 | 0 | 0 | 56 | 99 | 99 | 105 |
| 6 | 0 | 0 | 58 | 104 | 107 | 107 | 0 | 10 | 82 | 108 | 108 | 108 |
| **Mean** | 0 | 99 | 58 | 103 | 106 | 107 | 0 | 13 | 54 | 102 | 104 | 104 |
| **Max** | 0 | 35 | 97 | 106 | 107 | 108 | 0 | 60 | 90 | 108 | 108 | 108 |
| **Min** | 0 | 0 | 1 | 97 | 106 | 106 | 0 | 0 | 1 | 94 | 100 | 100 |
| **% RSD** | 0 | 157.8 | 71.2 | 3.4 | 0.5 | 0.7 | 0 | 174.9 | 59.3 | 5.4 | 3.5 | 3.3 |

## Claims

1. A pharmaceutical composition comprising:
*N*-((*S*)-5-((1*R*,2*S*)-2-(4-fluorophenyl)cyclopropylamino)-1-(4-methylpiperazin-1-yl)-1-oxopentan-2-yl)-4-(1*H*-1,2,3-triazol-1-yl)benzamide (Compound **A**), or a pharmaceutically acceptable salt thereof, and
at least one stabilizer chosen from citric acid, fumaric acid, and tartaric acid.

2. The pharmaceutical composition of claim 1, wherein Compound **A,** or a pharmaceutically acceptable salt thereof, is a tosylate salt of Compound **A.**

3. The pharmaceutical composition of claim 1, wherein Compound **A,** or a pharmaceutically acceptable salt thereof, is a ditosylate salt of Compound **A.**

4. The pharmaceutical composition of any one of claims 1 to 3, wherein Compound **A,** or the pharmaceutically acceptable salt thereof, is present in an amount of between about 2 and about 10% w/w, measured as the free base.

5. The pharmaceutical composition of any one of claims 1 to 3, wherein Compound **A,** or the pharmaceutically acceptable salt thereof, is present in an amount of between about 20 and about 30% w/w, measured as the free base.

6. The pharmaceutical composition of any one of claims 1 to 4, wherein the at least one stabilizer is present in an amount of between about 2 and about 10% w/w.

7. The pharmaceutical composition of any one of claims 1 to 3 or 5, wherein at least one stabilizer is present in an amount of between about 20 and about 30% w/w.

8. The pharmaceutical composition of any one of claims 1 to 7, wherein the composition comprises one or more fillers chosen from silicified microcrystalline cellulose, mannitol anhydrous lactose, and pre-gelatinized starch.

9. The pharmaceutical composition of claim 8, wherein the one or more fillers is present in the pharmaceutical composition in an amount of about 75 to about 90%.

10. The pharmaceutical composition of claim 8, wherein the one or more fillers is present in the pharmaceutical composition in an amount of about 35 to about 50%.

11. The pharmaceutical composition of any one of claims 1 to 10, wherein the composition comprises one or more disintegrants chosen from croscarmellose sodium Crospovidone XL and sodium starch glycolate

12. The pharmaceutical composition of claim 11, wherein the one or more disintegrants is present in the pharmaceutical composition in an amount between about 2 and about 10%.

13. The pharmaceutical composition of any one of claims 1 to 12, wherein the composition comprises one or more lubricants chosen from magnesium stearate sodium stearyl fumarate and stearic acid

14. The pharmaceutical composition of claim 13, wherein the one or more lubricants is present in the pharmaceutical composition in an amount between about 0.1 and about 1%.

15. The pharmaceutical composition of claim 1, wherein the composition comprises: 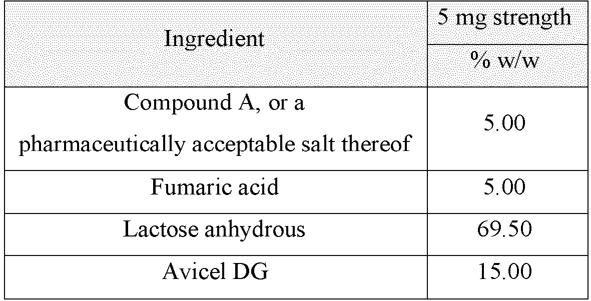 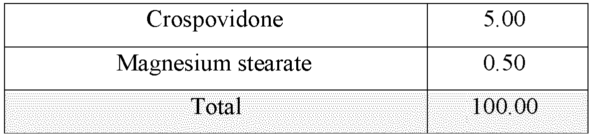 wherein Compound A is measured as the free base.

16. The pharmaceutical composition of claim 1, wherein the composition comprises: 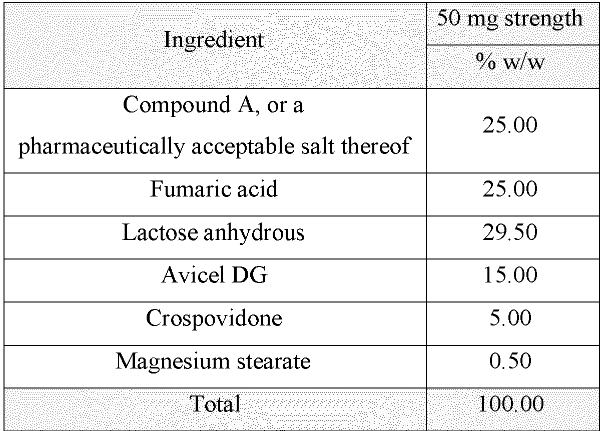 wherein Compound A is measured as the free base.

17. A pharmaceutical composition of any one of claims 1 to 15, for use in a method of treating cancer, myeloproliferative neoplasm, or inflammatory disease.

18. The pharmaceutical composition for use according to claim 17, wherein the cancer is Ewing's sarcoma, multiple myeloma, T-cell luekemia, Wilm's tumor, small-cell lung cancer, bladder cancer, prostate cancer, breast cancer, head / neck cancer, colon cancer, or ovarian cancer.

19. The pharmaceutical composition for use according to claim 17, wherein the myeloproliferative neoplasm is myelofibrosis, polycythemia vera, essential thrombocythemia, myelodysplastic syndrome (MDS), acute myelogenous leukemia (AML), or chronic myelogenous leukemia (CML).

20. The pharmaceutical composition for use according to claim 17, wherein the inflammatory disease is inflammatory bowel disease, rheumatoid arthritis, or systemic lupus erythematosus.

## Patentansprüche

1. Eine pharmazeutische Zusammensetzung, umfassend:
*N*-((*S*)-5-((1*R*,2*S*)-2-(4-Fluorphenyl)cyclopropylamino)-1-(4-methylpiperazin-1-yl)-1-oxopentan-2-yl)-4-(1*H*-1,2,3-triazol-1-yl)benzamid (Verbindung **A**) oder ein pharmazeutisch annehmbares Salz davon, und
wenigstens einen Stabilisator, ausgewählt aus Citronensäure, Fumarsäure und Weinsäure.

2. Die pharmazeutische Zusammensetzung nach Anspruch 1, wobei Verbindung **A** oder ein pharmazeutisch annehmbares Salz davon ein Tosylatsalz von Verbindung **A** ist.

3. Die pharmazeutische Zusammensetzung nach Anspruch 1, wobei Verbindung **A** oder ein pharmazeutisch annehmbares Salz davon ein Ditosylatsalz von Verbindung **A** ist.

4. Die pharmazeutische Zusammensetzung nach einem der Ansprüche 1 bis 3, wobei Verbindung A oder das pharmazeutisch annehmbare Salz davon in einer Menge zwischen etwa 2 und etwa 10 % Gew.-/Gew., gemessen als freie Base, vorliegt.

5. Die pharmazeutische Zusammensetzung nach einem der Ansprüche 1 bis 3, wobei Verbindung A oder das pharmazeutisch annehmbare Salz davon in einer Menge zwischen etwa 20 und etwa 30 % Gew.-/Gew., gemessen als freie Base, vorliegt.

6. Die pharmazeutische Zusammensetzung nach einem der Ansprüche 1 bis 4, wobei der wenigstens eine Stabilisator in einer Menge zwischen etwa 2 und etwa 10 % Gew./Gew. vorliegt.

7. Die pharmazeutische Zusammensetzung nach einem der Ansprüche 1 bis 3 oder 5, wobei der wenigstens eine Stabilisator in einer Menge zwischen etwa 20 und etwa 30 % Gew./Gew. vorliegt.

8. Die pharmazeutische Zusammensetzung nach einem der Ansprüche 1 bis 7, wobei die Zusammensetzung einen oder mehrere Füllstoffe umfasst, ausgewählt aus verkieselter mikrokristalliner Cellulose, mannitolwasserfreier Lactose und vorgelatinierter Stärke.

9. Die pharmazeutische Zusammensetzung nach Anspruch 8, wobei der eine oder die mehreren Füllstoffe in der pharmazeutischen Zusammensetzung in einer Menge von etwa 75 bis etwa 90 % vorliegen.

10. Die pharmazeutische Zusammensetzung nach Anspruch 8, wobei der eine oder die mehreren Füllstoffe in der pharmazeutischen Zusammensetzung in einer Menge von etwa 35 bis etwa 50 % vorliegen.

11. Die pharmazeutische Zusammensetzung nach einem der Ansprüche 1 bis 10, wobei die Zusammensetzung ein oder mehrere Sprengmittel umfasst, ausgewählt aus Croscarmellose-Natrium, Crospovidon XL und Natriumstärkeglycolat.

12. Die pharmazeutische Zusammensetzung nach Anspruch 11, wobei das eine oder die mehreren Sprengmittel in der pharmazeutischen Zusammensetzung in einer Menge zwischen etwa 2 und etwa 10 % vorliegen.

13. Die pharmazeutische Zusammensetzung nach einem der Ansprüche 1 bis 12, wobei die Zusammensetzung ein oder mehrere Schmiermittel umfasst, ausgewählt aus Magnesiumstearat, Natriumstearylfumarat und Stearinsäure.

14. Die pharmazeutische Zusammensetzung nach Anspruch 13, wobei das eine oder die mehreren Schmiermittel in der pharmazeutischen Zusammensetzung in einer Menge zwischen etwa 0,1 und etwa 1 % vorliegen.

15. Die pharmazeutische Zusammensetzung nach Anspruch 1, wobei die Zusammensetzung umfasst: 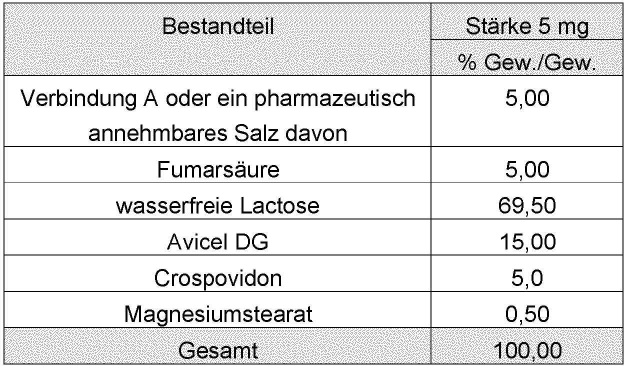 wobei Verbindung A als freie Base gemessen wird.

16. Die pharmazeutische Zusammensetzung nach Anspruch 1, wobei die Zusammensetzung umfasst: 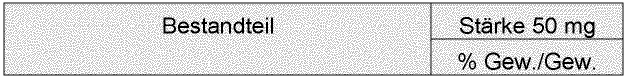 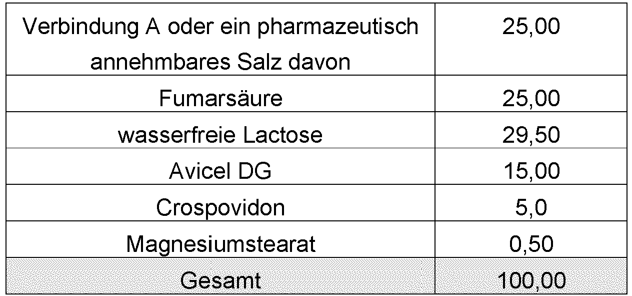 wobei Verbindung A als freie Base gemessen wird.

17. Eine pharmazeutische Zusammensetzung nach einem der Ansprüche 1 bis 15 zur Verwendung bei einem Verfahren zur Behandlung von Krebs, myeloproliferativer Neoplasie oder einer Entzündungserkrankung.

18. Die pharmazeutische Zusammensetzung zur Verwendung gemäß Anspruch 17, wobei der Krebs Ewing-Sarkom, multiples Myelom, T-Zell-Leukämie, Wilms-Tumor, kleinzelliges Lungenkarzinom, Blasenkrebs, Prostatakarzinom, Brustkrebs, Kopf-Hals-Tumor, Kolonkarzinom oder Ovarialkarzinom ist.

19. Die pharmazeutische Zusammensetzung zur Verwendung gemäß Anspruch 17, wobei die myeloproliferative Neoplasie Myelofibrose, Polycythemia vera, essentielle Thrombozythämie, myelodysplastisches Syndrom (MDS), akute myeloische Leukämie (AML) oder chronische myeloische Leukämie (CML) ist.

20. Die pharmazeutische Zusammensetzung zur Verwendung gemäß Anspruch 17, wobei die Entzündungserkrankung entzündliche Darmerkrankung, rheumatoide Arthritis oder systemischer Lupus erythematodes ist.

## Revendications

1. Composition pharmaceutique comprenant :
du *N*-((*S*)-5-((1*R*,2*S*)-2-(4-fluorophényl)cyclopropylamino)-1-(4-méthylpipérazin-1-yl)-1-oxopentan-2-yl)-4-(1*H*-1,2,3-triazol-1-yl)benzamide (Composé A), ou un sel pharmaceutiquement acceptable de celui-ci, et
au moins un stabilisant choisi parmi l'acide citrique, l'acide fumarique et l'acide tartrique.

2. Composition pharmaceutique selon la revendication 1, dans laquelle le composé A, ou un sel pharmaceutiquement acceptable de celui-ci, est un sel de tosylate du composé A.

3. Composition pharmaceutique selon la revendication 1, dans laquelle le composé A, ou un sel pharmaceutiquement acceptable de celui-ci est un sel de ditosylate du composé A.

4. Composition pharmaceutique selon l'une quelconque des revendications 1 à 3, dans laquelle le composé A, ou le sel pharmaceutiquement acceptable de celui-ci, est présent en une quantité comprise entre environ 2 et environ 10 % en poids (P/P), mesuré sous forme de base libre.

5. Composition pharmaceutique selon l'une quelconque des revendications 1 à 3, dans laquelle le composé A, ou le sel pharmaceutiquement acceptable de celui-ci, est présent en une quantité comprise entre environ 20 et environ 30 % en poids (P/P), mesuré sous forme de base libre.

6. Composition pharmaceutique selon l'une quelconque des revendications 1 à 4, dans laquelle l'au moins un stabilisant est présent en une quantité comprise entre environ 2 % et environ 10 % P/P.

7. Composition pharmaceutique selon l'une quelconque des revendications 1 à 3 ou 5, dans laquelle l'au moins un stabilisant est présent en une quantité comprise entre environ 20 % et environ 30 % P/P.

8. Composition pharmaceutique selon l'une quelconque des revendications 1 à 7, dans laquelle la composition comprend une ou plusieurs charges choisies parmi la cellulose microcristalline silicifiée, le mannitol, le lactose anhydre et l'amidon prégélatinisé.

9. Composition pharmaceutique selon la revendication 8, dans laquelle les une ou plusieurs charges sont présentes dans la composition pharmaceutique en une quantité d'environ 75 % à environ 90 %.

10. Composition pharmaceutique selon la revendication 8, dans laquelle les une ou plusieurs charges sont présentes dans la composition pharmaceutique en une quantité d'environ 35 % à environ 50 %.

11. Composition pharmaceutique selon l'une quelconque des revendications 1 à 10, dans laquelle la composition comprend un ou plusieurs délitants choisis parmi la croscarmellose sodique, la crospovidone XL et le glycolate d'amidon de sodium.

12. Composition pharmaceutique selon la revendication 11, dans laquelle les un ou plusieurs délitants sont présents dans la composition pharmaceutique en une quantité comprise entre environ 2 et environ 10 %.

13. Composition pharmaceutique selon l'une quelconque des revendications 1 à 12, dans laquelle la composition comprend un ou plusieurs lubrifiants choisis parmi le stéarate de magnésium, le stéaryl fumarate de sodium et l'acide stéarique.

14. Composition pharmaceutique selon la revendication 13, dans laquelle les un ou plusieurs lubrifiants sont présents dans la composition pharmaceutique en une quantité comprise entre environ 0,1 et environ 1 %.

15. Composition pharmaceutique selon la revendication 1, dans laquelle la composition comprend : 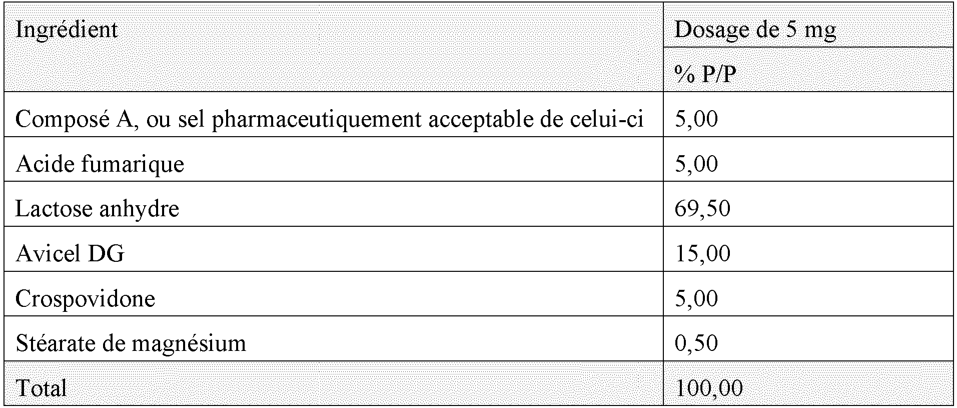 dans laquelle le composé A est mesuré sous forme de base libre.

16. Composition pharmaceutique selon la revendication 1, dans laquelle la composition comprend : 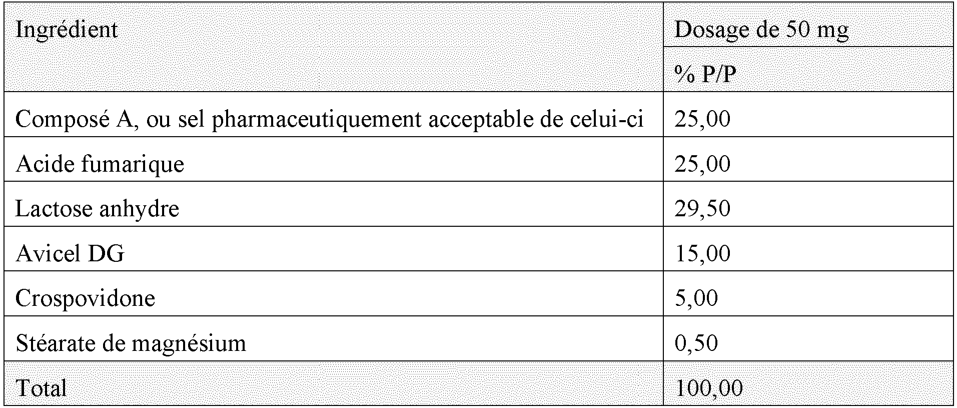 dans laquelle le composé A est mesuré sous forme de base libre.

17. Composition pharmaceutique selon l'une quelconque des revendications 1 à 15, pour utilisation dans une méthode de traitement du cancer, d'un néoplasme myéloprolifératif, ou d'une maladie inflammatoire.

18. Composition pharmaceutique pour utilisation selon la revendication 17, où le cancer est un sarcome d'Ewing, un myélome multiple, une leucémie des cellules T, une tumeur de Wilms, un cancer du poumon à petites cellules, un cancer de la vessie, un cancer de la prostate, un cancer du sein, un cancer de la tête / du cou, un cancer du côlon ou un cancer de l'ovaire.

19. Composition pharmaceutique pour utilisation selon la revendication 17, où le néoplasme myéloprolifératif est une myélofibrose, une polyglobulie essentielle, une thrombocytémie essentielle, un syndrome myélodysplasique (MDS), une leucémie myéloïde aiguë (AML) ou une leucémie myéloïde chronique (CML).

20. Composition pharmaceutique pour utilisation selon la revendication 17, où la maladie inflammatoire est une maladie inflammatoire de l'intestin, une polyarthrite rhumatoïde ou un lupus érythémateux systémique.
